(19) Europäisches Patentamt
European Patent Office
Office européen des brevets

(11) EP 1 598 038 A1

(12) **EUROPEAN PATENT APPLICATION**
published in accordance with Art. 158(3) EPC

(43) Date of publication:
23.11.2005 Bulletin 2005/47

(51) Int Cl.7: **A61F 13/538**, A61F 13/472, A61F 13/534, A61F 13/535

(21) Application number: 04711729.6

(22) Date of filing: 17.02.2004

(86) International application number:
PCT/JP2004/001729

(87) International publication number:
WO 2004/073573 (02.09.2004 Gazette 2004/36)

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 18.02.2003 JP 2003040369

(71) Applicant: UNI-CHARM CORPORATION
Kawanoe-shi Ehime 799-0111 (JP)

(72) Inventors:
• MIZUTANI, Satoshi,
Techn. Center, Uni-Charm Corp.
Mitoyo-gun, Kagawa 7691602 (JP)
• NODA, Yuki, Technical Center, Uni-Charm Corp.
Mitoyo-gun, Kagawa 7691602 (JP)

(74) Representative: McNally, Roisin et al
Murgitroyd & Company
165-169 Scotland Street
Glasgow G5 8PL (GB)

(54) **INTER-LABIUM PAD**

(57) An object of the present invention is to provide an interlabial pad, with which an absorbent body does not become lopsided while the interlabial pad is fitted on and yet with which it is unlikely for a foreign-object sensation to be felt while the interlabial pad is fitted on. The present invention provides an interlabial pad comprising: an absorbent body, absorbing liquids; and a cover body, covering the abovementioned absorbent body in an enclosing manner; and wherein the abovementioned absorbent body comprises fiber aggregates in which the fiber directions are oriented randomly, and with the fiber aggregates, the flexural rigidities as Gurley bending resistances are in the range from 25 mg to 130 mg and the ratio of flexural rigidities in two mutually orthogonal directions is in the range from 0.5 to 2.0.

**FIG. 1**

EP 1 598 038 A1

## EP 1 598 038 A1

**Description**

**FIELD OF THE INVENTION**

The present invention relates to an interlabial pad to be fitted between a woman's labia. More specifically, the present invention relates to an interlabial pad that is improved in fitting properties and fitting comfort.

**RELATED ART**

Interlabial pads have been known as menstrual products of an intermediate positioning between menstrual napkins and tampons. An interlabial pad is partially put between a woman's labia and fitted so as to be in contact with the inner labial walls. Since an interlabial pad is more closely fitted to the body in comparison to a menstrual napkin, it prevents the leakage of menstrual blood and prevents the menstrual blood from dispersing and coming in contact with a wide area of the body and is thus sanitary and clean. Also due to being more compact than a menstrual napkin, an interlabial pad is comfortable, excellent in fitting sensation, and low in the psychological resistance in the fitting process in comparison to a tampon that is inserted inside the vagina.

As such an interlabial pad, the present inventors have proposed an interlabial pad comprising a liquid permeable surface side sheet, a liquid impermeable back face sheet, an absorbent body disposed between the surface side sheet and the back face sheet, and a mini-sheet piece attached to the surface at one side of the back face sheet (International Patent Publication Pamphlet No. 02/094148). The back face sheet of this interlabial has two surfaces, that is, an inner surface at which the absorbent body is positioned and an outer surfaces, and the mini-sheet piece is attached to the outer surface which is opposite to the inner surface to form a pocket into which a finger can be inserted and the shape of the entrance of this pocket is made a shape that is flat in the planar direction of the sheet.

Thus in fitting on this interlabial pad, a ball of a finger becomes inserted smoothly along the surface of the back face sheet and into the pocket, and the interlabial pad can be fitted readily at an appropriate location between the labia. Erroneous fitting can thus be reduced significantly with the above-described interlabial pad. The attachment of menstrual blood onto the fingertip in the fitting process can also be prevented.

Besides the above-described interlabial pad, there is an interlabial pad (trade name: "Envive (Moderate)"), which has been test-sold in the USA by The Procter and Gamble Corp. (referred to hereinafter as "PG Corp."). This interlabial pad is an interlabial pad comprising a liquid permeable surface side sheet, a liquid impermeable back face sheet, an absorbent body disposed between the surface side sheet and the back face sheet, and a semicircular holding part for fitting the interlabial pad between the labia disposed on the back face side of the interlabial pad.

This holding part is formed by embossing the back face sheet and the absorbent body at the same time, moves along with the main body of the interlabial pad, and has rigidity. Thus when a wearer holds the holding part of the interlabial pad with fingers and guides the pad between the labia, the positional relationship between the main body and the holding part does not deviate and the wearer can fit the interlabial pad on at an intended position.

Also, the degree of elongation in a lateral direction of the absorbent body is made high for this interlabial pad in order to prevent rupture due to embossing in the process of embossing the back face sheet and the absorbent body at the same time to form the holding part. Specifically, a density of a fiber aggregate that forms the absorbent body is made approximately 0.048g/cm$^3$ to make flexibility among fibers high and a fiber orientation is directed mainly in a longitudinal direction to increase the degree of elongation in the lateral direction of the absorbent body.

Furthermore, there is an interlabial pad (trade name: "Fresh'n Fit Padette" (or INSYNC)), sold by A-Fem Medical Corporation. This interlabial pad has an absorbent body which is restrained in the flexibility among fibers by orienting the fibers of a fiber aggregate which forms the absorbent body mainly in the longitudinal direction of the absorbent body and furthermore by setting the density of the fiber aggregate to be approximately 0.097g/cm$^3$. The absorbent body has a flexural rigidity (Gurley bending resistance) of approximately 368 mg in the longitudinal direction and approximately 124 mg in the lateral direction that is orthogonal to the longitudinal direction of the absorbent body and is thus high in flexural rigidity. The interlabial pad can open labia which deform freely when the interlabial pad is fitted between the labia and the interlabial pad can thus be fitted into deeper parts of the labia by making the lexural rigidity of the interlabial pad high.

The labia are stretched by sudden changes in body orientation and the labial inner walls and the vestibular floor also become stretched accordingly. Thus in order to prevent a wearer of an interlabial pad from feeling a foreign-object sensation when the labia are stretched, the bendability of the absorbent body which occupies a large part of the interlabial pad must be made extremely high and thus enabled to match the labia stretching.

For example, the absorbent member of the abovementioned interlabial pad "Envive (Moderate)" of PG Corp. having a tensile elongation of 15%, and a flexural rigidity as Gurley bending resistance of approximately 33 mg in the longitudinal direction and approximately 14 mg in the lateral direction is extremely high in bendability, and deforms readily in accordance with the inner labial walls that deform in accompaniment with the movements of the wearer and is thus unlikely to make a wearer feel a foreign-object sensation.

However, since the flexibility of the absorbent body is made too high, the absorbent body deforms as much as or more than the deformation of the labial inner walls that occur when a wearer moves and the absorbent body thus becomes lopsided with regard to the PG Corp.'s interlabial pad "Envive (Moderate)". In particular, since the fibers are mainly oriented in the longitudinal direction with regard to this interlabial pad, the ratio of the flexural rigidities of the longitudinal direction and the lateral direction takes on a high value of 2.4 and there is thus a high possibility for the absorbent body to become lopsided in the lateral direction in which the flexural rigidity is low. When the absorbent body becomes lopsided, the bulk of the absorbent body becomes high at parts and the area contacting the inner labial walls and the interlabial pad decreases, increasing the likelihood of the falling off of the interlabial pad from between the labia and causing the wearer to feel a foreign-object sensation.

In the case of the interlabial pad "INSYNC" sold by A-Fem Medical Corporation, since an absorbent body that is reduced in the flexibility among fibers is used, the tensile elongation is 25% in the longitudinal direction and the stretching property is high. However, since this interlabial pad is also high in tensile strength, it is extremely high in flexural rigidity and thus makes a wearer feel a foreign-object sensation. Also, in the case of this interlabial pad, since the fibers are mainly oriented in the longitudinal direction, the ratio of flexural rigidities of the longitudinal direction and the lateral direction takes a high value of 3.0, and thus, as with the interlabial pad of PG Corp., there is a high likelihood for the absorbent body to become lopsided. Since the interlabial pad "INSYNC" can furthermore be fitted deeply between the labia, there is the possibility of damaging the inner labial walls.

Thus with the prior-art interlabial pads, it was difficult to prevent the absorbent body from becoming lopsided while an interlabial pad is fitted on and yet make it unlikely for a foreign-object sensation to be felt while the interlabial pad is fitted on.

## SUMMARY OF THE INVENTION

The present invention has been made to resolve the above issue and an object thereof is to provide an interlabial pad which has an absorbent body not becoming lopsided when the interlabial pad is fitted on and yet is unlikely for a foreign-object sensation to be felt while the interlabial pad is fitting on.

The present invention has been made based on the finding that an absorbent body which makes up an interlabial pad can be prevented from becoming lopsided in one direction and yet be made unlikely to make a wearer feel a foreign-object sensation by controlling the flexural rigidity of the absorbent body in the longitudinal direction to be substantially equal to the flexural rigidity in the lateral direction.

Specifically, the present invention provides the following.

(1) An interlabial pad comprising: an absorbent body absorbing liquid; and a cover body covering the absorbent body in an enclosing manner; wherein the absorbent body comprises a fiber aggregate in which fiber directions are oriented randomly; and

the fiber aggregate has a flexural rigidity as Gurley bending resistance being in a range from 25 mg to 130 mg and a ratio of flexural rigidities in two mutually orthogonal directions being in a range from 0.5 to 2.0.

According to the present invention, the fiber orientation of the fiber aggregates forming the absorbent body are made random and a flexural rigidity of the fiber aggregates in an arbitrary direction is made substantially the same as another flexural rigidity of the fiber aggregates in the direction orthogonal to this arbitrary direction. The absorbent body is thereby made less likely to become lopsided in one direction. Since the bulk of the absorbent body can thus be prevented from changing at parts, the falling off of the interlabial pad due to lowering of the area contacting with the inner labial walls can be prevented and a wearer can be prevented from feeling a foreign-object sensation.

Also, by setting the flexural rigidities (Gurley bending resistances) of the fiber aggregates to be in the range of 25 mg to 130 mg and preferably 35 mg to 90 mg, a wearer can be prevented from feeling a foreign-object sensation and the absorbent body can be prevented from becoming lopsided due to the absorbent body deforming as much or more than the inner labial walls deforming. When a flexural rigidity of the fiber aggregates is greater than 130 mg, it becomes difficult for various movements of the labial inner walls to be followed and a wearer is made to feel a foreign-object sensation. Also, gaps form between the labial inner walls and the interlabial pad may leak body fluids. On the other hand, when a flexural rigidity of the fiber aggregates is less than 25 mg, the absorbent body deforms by as much or more than the deformation of the labial inner walls and becomes lopsided, the bulk of the absorbent body becomes different at parts, and a wearer is made to feel a foreign-object sensation.

The flexural rigidities of the fiber aggregates can be controlled by controlling mainly the maximum tensile elongation and apparent density of the fiber aggregates. The flexural rigidities of the fiber aggregates can also be controlled by adjusting the fiber lengths of the fibers that make up the fiber aggregates. The flexural rigidities of a fiber aggregate are preferably adjusted to be within the predetermined ranges by forming the fiber aggregates from fibers having an average fiber length of 10 to 51 mm and preferably 25 to 50 mm and adjusting the maximum tensile elongation and apparent density of the fiber aggregates to be within appropriate ranges.

With regard to the fibers that form the fiber aggregates, the longer the fiber length, the more readily the fibers become entangled with each other. Thus when the fiber aggregates are stretched, the spatial shapes between the fibers of the fiber aggregates vary, the entangled fibers become loosened from each other, and the maximum tensile elongation of the fiber aggregates becomes high. When the maximum tensile elongation is high, even when the vestibular floor undergoes a stretching movement, the fiber aggregates can match the movement without separating. However, a maximum tensile elongation that is too high is not preferable as the absorbent body will then deform excessively and become lopsided.

Meanwhile, when the average fiber length of the fibers is too short, the maximum tensile elongation of the absorbent body will be small, the absorbent body will not stretch according to the movements of a wearer, the fiber aggregates will separate, and lumps of fibers will become overlapped. The bulk of the absorbent body will differ at parts, causing the area contacting with the inner labial walls to decrease and the percentage of fall-off to increase.

Also, as the average fiber length of the fibers that form the fiber aggregates becomes longer, since the points of mutual entanglement of fibers increase and the distance between fibers becomes less likely to be no less than the fineness, the apparent density of the absorbent body tends to become low. Spaces between fibers thus increase and the degree of flexibility of the fiber aggregates increases. However, it is not preferable for the fiber length to become excessively long since the flexibility of the fiber aggregates will then become too high, causing the absorbent body to become lopsided and the percentage of falling off of the interlabial pad to increase.

Meanwhile, as the average fiber length of the fibers that form the fiber aggregates becomes shorter, the points of mutual entanglement of fibers decrease and the apparent density of the absorbent body tends to become high. Spaces between fibers thus decrease, the flexibility of the fiber aggregates decreases, and this is not favorable since a wearer is made more likely to feel a foreign-body sensation and the inner labial walls may become damaged.

Also, the ratio of a flexure rigidity of the fiber aggregates in a direction (an arbitrary direction) to a flexure rigidity in another direction which is orthogonal to said direction, for example, the ratio of the flexural rigidities in the length direction of the absorbent body (hereinafter called "MD direction") to the flexural rigidities in the width direction of the absorbent body (hereinafter called "CD direction") that is orthogonal to the MD direction (hereinafter, this ratio shall be called "MD/CD ratio of flexural rigidity") is preferably set in the range of 0.5 to 2.0 and more preferably in the range of 0.7 to 1.5. When the MD/CD ratio of flexural rigidity is in the abovementioned range, the values of the flexural rigidities of mutually orthogonal directions will be substantially the same. In this case, the absorbent body is unlikely to become lopsided in one direction and the bulk of the absorbent body can be prevented from changing at parts. Therefore, the area contacting the interlabial pad with the inner labial walls can be prevented from declining, the interlabial pad can be prevented from falling off, and the making of a wearer feel a foreign-object sensation can be prevented.

When the MD/CD ratio of flexural rigidity is outside the range of 0.5 to 2.0, the absorbent body tends to bend readily in one direction and become lopsided readily.

The MD/CD ratio of flexural rigidity can be adjusted by randomly orienting the fibers of the fiber aggregates. Though the MD/CD ratio of flexural rigidity is also affected by the patterning such as embossing, slitting, etc., of the absorbent body, it is important that the fibers is oriented randomly so that each and every fiber can move without causing lopsidedness. The ratio of the maximum tensile strength in the longitudinal direction of the fiber aggregate to the maximum tensile strength in the lateral direction (referred to hereinafter as "MD/CD ratio of maximum tensile strength") can be used as an index for expressing the randomness of fiber orientation. Specifically, the MD/CD ratio of maximum tensile strength is preferably in the range of 0.5 to 2.5.

The flexural rigidity refers to the Gurley bending resistance as measured by a Gurley stiffness tester. An arbitrary direction of a fiber aggregate does not indicate a specific direction of the fiber aggregate, such as the longitudinal direction or the lateral direction, etc., and two mutually orthogonal directions do not always refer to the longitudinal direction and the lateral direction. However, when a fiber aggregate is molded in a continuous manner, the molding direction (referred to hereinafter as the "MD direction") which is the direction in which the fiber aggregate is continuous becomes the longitudinal direction of the fiber aggregate, and since the lateral direction is orthogonal to this longitudinal direction (MD direction), for the sake of convenience, an arbitrary direction of a fiber aggregate shall refer to the longitudinal direction (MD direction) and the direction orthogonal to the arbitrary direction (referred to hereinafter as the "CD direction")shall refer to the lateral direction (or CD direction).

(2) The interlabial pad according to (1); wherein flexural rigidities in two mutually orthogonal directions of the fiber aggregate are substantially the same.

According to the present invention, since the values of mutually orthogonal flexural rigidities are substantially the same, the absorbent body is unlikely to become lopsided in one direction and the bulk of the absorbent body is prevented from varying at parts while the interlabial pad is fitted on. The area contacting the interlabial pad with the inner labial walls can be prevented from declining, and the falling off of the interlabial pad and the making of a wearer feel a foreign-object sensation can thus be prevented.

(3) The interlabial pad according to (1) or (2); wherein the absorbent body is formed by layering the fiber aggregate and another fiber aggregate that differ each other in tensile elongation; and one of the fiber aggregates which is positioned at a vestibular floor side when the interlabial pad is fitted between labia has a higher tensile elongation than that of the other fiber aggregate which is positioned at a side opposite to the vestibular floor side.

According to the present invention, the fiber aggregates that are positioned at the vestibular floor side (the upper region to be described later) stretch and match the movements of the body, when the interlabial pad is fitted between labia. Thus especially the vestibular floor, which is hypersensitive, is unlikely to be irritated and a wearer is unlikely to feel a foreign-object sensation. Meanwhile, in the fiber aggregates positioned at the side opposite to the vestibular floor side (the lower region to be described later), that is, the side opposite to the side positioned at the vestibular floor, the spatial shapes between fibers can prevent the absorbent body from deforming regardless of movements of the body and readily hold body fluids that have been captured once between the fibers. That is, According to the present invention, at the upper region that is in contact with the vestibular floor, the absorbent body can deform in accordance with the body, and at the lower region, the absorbent body can hold menstrual blood. Also, even if the fiber aggregates at the lower region separate and lumps of the fiber aggregates overlap, since this part is not in contact with the vestibular floor, it is unlikely to irritate the vestibular floor.

(4) The interlabial pad according to (3); wherein the fiber aggregate positioned at the vestibular floor side has a tensile elongation of 60% or more than that in a dry state even in a wet state in which liquids are absorbed.

According to the present invention, the fibers that form the absorbent body can be prevented from being drawn to each other due to the surface tension of menstrual blood and thereby causing the distance between materials to narrow and the thickness of the absorbent body to be thinned when the absorbent body has absorbed menstrual blood. When the thickness of the interlabial pad becomes thin, the repulsive force that opposes the holding force of the labia weakens and the possibility of the interlabial pad falling off from the labia increases. However, According to the present invention, since the interlabial pad can be prevented from becoming thin, the interlabial pad can be prevented from falling off. Also, when the maximum tensile elongation in the wet state is less than 60% of the maximum tensile elongation in the dry state, the flexibility among fibers decreases extremely and the density of the absorbent body increases. Though the spaces between fibers thus deform less readily and a wearer is made to feel a foreign-object sensation, According to the present invention, a wearer can be prevented from feeling a foreign-object sensation.

Here, the wet state refers to a state in which menstrual blood and other body fluids are absorbed and refers to a state below the absorption amount (absorption capacity) of the absorbent body.

(5) The interlabial pad according to (3) or (4); wherein the fiber aggregate positioned at the side opposite to the vestibular floor side comprises another fiber aggregate that differ in tensile elongation; and one of the fiber aggregates which is positioned at the vestibular floor side has a higher tensile elongation than that of the other fiber aggregate which is positioned at the side opposite to the vestibular floor side.

An interlabial pad tends to fall off since external impacts, due to a napkin that is used in combination, etc., are applied readily to the side (the lowermost region to be described later) opposite to the vestibular floor side of the fiber aggregates positioned at the side opposite to the vestibular floor side, which tend to protrude out from between the labia. However, by making the flexibility among fibers of this part higher than that of the fiber aggregates at the lower region as in the present invention, the external impacts can be cushioned and the falling off of the interlabial pad can be prevented.

(6) The interlabial pad according to (1) or (2); wherein the interlabial pad is a substantially planar interlabial pad; and the cover body that covers the absorbent body comprises a liquid permeable surface side sheet and a liquid impermeable back face sheet; and wherein the absorbent body is formed by layering the fiber aggregate and another fiber aggregate that differ each other in tensile elongation; and one of the fiber aggregates which is positioned at the vestibular floor side has a higher tensile elongation than that of the other fiber aggregate which is positioned at the side opposite to the vestibular floor side.

According to the present invention, when the interlabial pad is fitted upon being folded along a length direction central line so that the back face sheet faces to each other, the fiber aggregates having a high flexibility among fibers are put in contact not only with the vestibular floor but also with the inner labial walls. Stretching movements and variations of the inner labial walls can thus be followed better.

(7) The interlabial pad according to (6); wherein a proportion of the fiber aggregate having the higher tensile elongation and a proportion of the fiber aggregate having the lower tensile elongation are substantially the same in the thickness direction of the absorbent body

(8) The interlabial pad according to (6); wherein a proportion of the fiber aggregate having the higher tensile elongation is larger than a proportion of the fiber aggregate having the lower tensile elongation in the thickness direction of the absorbent body at a vicinity of a longitudinal direction central line.

According to the present invention, when the interlabial pad is fitted upon being folded along a longitudinal direction central line so that the back face sheet faces to each other, the weight proportion of the fiber aggregates having the high tensile elongation is high and the tensile elongation is thus high at the upper region in contact with the vestibular floor. The part in contact with the vestibular floor thus stretches readily and can better follow the stretching of the vestibular floor. Also, even when an external pressure that pushes upwards from below is applied as when a wearer sits down on a chair, etc., the stiff sensation of the fiber aggregates of low tensile elongation is cushioned by the fiber aggregates of the upper region, thus preventing the wearer from feeling discomfort. Furthermore in the lower region, the weight proportions of the fiber aggregates having the high tensile elongation to the fiber aggregates having the low tensile elongation are substantially equal, thus enabling the menstrual blood captured by the absorbent body to be held without becoming discharged.

(9) The interlabial pad according to (8); wherein the absorbent body comprises the fiber aggregate having the higher tensile elongation at outer peripheral parts and being positioned the entire thickness direction.

The vicinities of the outer peripheral edges of an absorbent body tend to protrude out from between the labia when an interlabial pad is fitted on. However, according to the present invention, since the fiber aggregates having the high tensile elongation is disposed across the entire thickness direction, when an external impact, due to a napkin used in combination, etc., is applied, the external impact can be cushioned by the fiber aggregates positioned at the outer peripheral parts and the interlabial pad can be prevented from falling off.

(10) The interlabial pad according to any one of (6) to (9); wherein the tensile elongation of the fiber aggregate having the higher tensile elongation is maintained at 60% or more than that in the dry state even in the wet state in which liquids are absorbed.

According to the present invention, the tensile elongation is maintained at no less than 60% of the tensile elongation in the dry state, the flexibility among fibers is not lowered extremely and the spaces between fibers are not readily deformed, even when the absorbent body absorbs menstrual blood and is put in the wet state. A wearer will therefore not be subject to discomfort due to the occurrence of a stiff sensation, etc.

(11) The interlabial pad according to any one of (6) to (10), wherein a dividing region which divides the absorbent body is provided at least substantially along the longitudinal direction central line at a rear of the absorbent body.

According to the present invention, a dividing region that divides the absorbent body is provided so that a repulsive force against bending will not act readily at the dorsal side of the interlabial pad. Thus at the dorsal side of the labia, the labia will not be spread more than necessary by the repulsive force of the interlabial pad, and the hypersensitive ostium vaginae or parts deeper than the ostium vaginae will be less likely to be irritated and the possibility of making a wearer feel a foreign-object sensation is reduced. Since a repulsive force against bending acts at the ventral side of the interlabial pad and the force folding the interlabial at the front of the labia and the repulsive force of the interlabial pad are balanced or the repulsive force of the interlabial pad is slightly greater, the interlabial pad can be prevented from falling off from the labia. Here, the dorsal side of the absorbent body refers to the direction in which the interlabial pad contacts the ostium vaginae side of the labia when it is fitted on.

## BRIEF DESCRIPTION OF THE DRAWINGS

Fig. 1 is a perspective sectional view of an interlabial pad of a first embodiment of the present invention.
Fig. 2 is a diagram for explaining a method for orienting fibers randomly.
Fig. 3 is a diagram showing the state in which an interlabial pad of a second embodiment of the present invention is fitted between labia.
Fig. 4 is a diagram showing the state in which an interlabial pad of a third embodiment of the present invention is fitted between labia.
Fig. 5A is a perspective sectional view of an interlabial pad of a fourth embodiment of the present invention.
Fig. 5B is a diagram showing the state in which the interlabial pad of the fourth embodiment of the present invention is fitted between labia.
Fig. 6A is a perspective sectional view of an interlabial pad of a fifth embodiment of the present invention.
Fig. 6B is a diagram showing the state in which the interlabial pad of the fifth embodiment of the present invention is fitted between labia.
Fig. 7A is a perspective sectional view of an interlabial pad of a sixth embodiment of the present invention.
Fig. 7B is a diagram showing the state in which the interlabial pad of the sixth embodiment of the present invention is fitted between labia.
Fig. 8A is a perspective sectional view of an interlabial pad of a seventh embodiment of the present invention.
Fig. 8B is a diagram showing the state in which the interlabial pad of the seventh embodiment of the present invention is fitted between labia.

Fig. 9A is a perspective sectional view of an interlabial pad of an eighth embodiment of the present invention.

Fig. 9B is a diagram showing the state in which the interlabial pad of the eighth embodiment of the present invention is fitted between labia.

Fig. 10 is a diagram showing an interlabial pad used for measuring the flexural rigidity.

## DESCRIPTIONS OF THE PREFERRED EMBODIMENT

Interlabial pads according to the present invention shall be described with reference to the drawings. In the description that follows, same members shall be provided with the same symbols and description thereof shall be omitted or simplified.

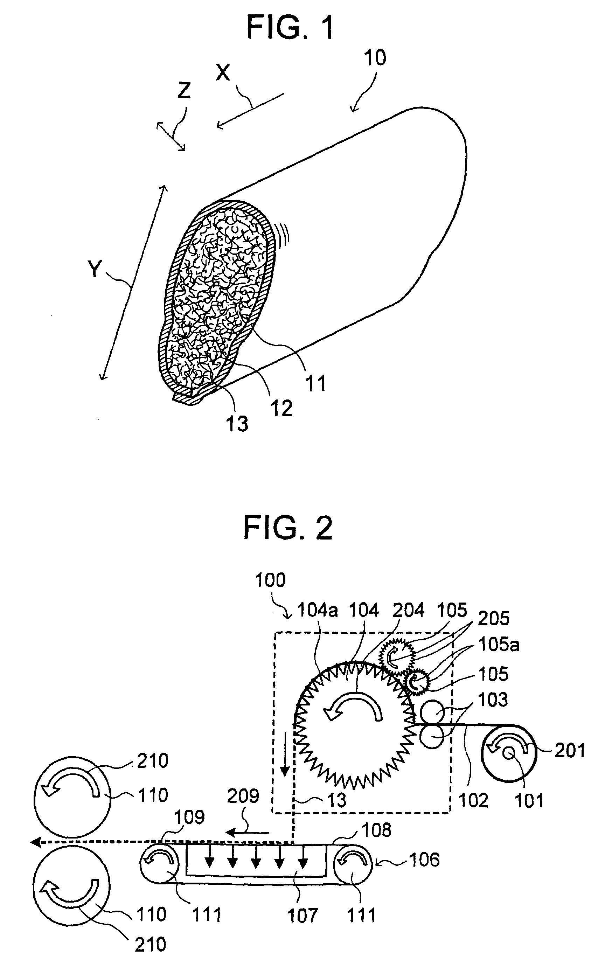

Fig. 1 is a perspective sectional view showing an interlabial pad 10 of a first embodiment according to the present invention. The interlabial pad 10 comprises an absorbent body 12 which absorbs liquids, and a cover body 11 which covers the absorbent body in an enclosing manner. The interlabial pad 10 has a substantially elliptical shape as the cross-sectional shape when sectioned in the thickness direction Y. The shape of the interlabial pad 10 is not restricted in particular as long as the shape enables the interlabial pad 10 to be sandwiched and held between labia, and the cross-sectional shape may be a rectangular shape, gourd-like shape, or teardrop shape, etc, for example.

The absorbent body 12 comprises a fiber aggregate which is formed of fibers 13 having a long fiber length being oriented randomly. A flexural rigidity as the Gurley bending resistance of said fiber aggregate are in the range from 25 to 130 mg and more preferably from 35 mg to 90 mg, and a ratio of the flexural rigidity in the longitudinal direction X and the flexural rigidity in the orthogonal lateral direction Z is from 0.5 to 2.0 and more preferably from 0.7 to 1.5.

When the flexural rigiditie (Gurley bending resistance) of the fiber aggregate is controlled to be in the range from 25 mg to 130 mg, a wearer is less likely to feel a foreign-object sensation and the fiber aggregate can be prevented from changing shape as much or more than inner labial walls change shape, thus the fibers 13 can be prevented from becoming lopsided. Also, when a ratio of the flexural rigidity in the longitudinal direction X of the fiber aggregate to the flexural rigidity in the lateral direction Z (MD/CD ratio of flexural rigidity) is in a range of 0.5 to 2.0, the values of the flexural rigidities in the longitudinal direction X and the lateral direction Z will be controlled to be substantially uniform and the absorbent body 12 will be less likely to become lopsided in one direction while the interlabial pad 10 is fitted between labia. The absorbent body 12 can thus be prevented from deforming at parts, and an area of the interlabial pad 10 contacting the inner labial walls may maintain, the interlabial pad 10 can be prevented from being fallen, and a foreign-object sensation to a wearer can be lessened. The MD/CD ratio of flexural rigidity of the fiber aggregate can be made small by orienting the fibers 13 randomly.

The fiber aggregate that forms the absorbent body 12 is an aggregate of solitary or mixed fibers 13 such as rayon, acetate, natural cotton, super absorbent polymer fibers, synthetic fibers, etc., and the fibers 13 have an average fiber length in a range from 10 to 51 mm and more preferably from 25 to 50 mm.

A method for orienting the fibers 13 having a long fiber length randomly shall be described with reference to Fig. 2. Fig. 2 is a diagram for explaining a method for molding a fiber aggregate that is to become the absorbent body 12. In the Figure, 102 is a fiber aggregate sheet in which fibers are accumulated and the fiber aggregate sheet 102 is wound around a winding roller 101.

By the rotation of a pair of feeding rollers 103, a winding roller 101 is rotated in the direction of a fiber shredder 100 (direction indicated by an arrow 201) and the fiber aggregate sheet 102 is thereby conveyed to the fiber shredder 100. This fiber shredder 100 has a garnet cutter roller 104, with which a plurality of cutting blades 104a are aligned so as to surround the circumference, and the fiber aggregate sheet 102 is cut up finely and fibrillated into the fibers 13 by this cutter roller 104. In order to fibrillate the fiber aggregate sheet 102 more finely, it is preferable to provide a plurality of small cutter rollers 105, each having the same structure as the cutter roller 104 and being smaller than the cutter roller 104. Each small cutter roller 105 rotates in the opposite direction (the direction indicated by an arrow 205) of the rotation direction (direction indicated by an arrow 204) of the cutter roller 104 and cutting blades 105a of each small cutter roller are set so as to be engaged with the cutting blades 104a of the cutter roller. This fiber shredder 100 is not limited to a garnet type cutter and may instead be equipped with a hammer mill type cutter.

By the rotation of the cutter roller 104 in the direction of a conveyor device 106 (the direction indicated by arrow 204), the fibers 13 obtained by fibrillating the fiber aggregate sheet 102, are conveyed onto a conveyor belt 108 provided in the conveyor device 106.

The conveyor device 106 is equipped with a conveyor belt 108 being annular and a pair of drive rollers 111 that turn the conveyor belt 108, which is spanned across the rollers. The rotation speed of the drive rollers 111 is preferably adjusted so that the conveying speed by the conveyor belt 108 will be 20 to 200 m/min. The conveyor belt 108 is a mesh type belt and a suction device 107 is equipped at the inner side of the conveyor belt 108 for attaching the fibers 13 onto the conveyor belt 108 by suction.

The fibers 13 are sucked by the suction device 107 and collected onto the conveyor belt 108 at the same time as they are separating from the cutter blade 104a of the cutter roller. The fibers 13 are sucked by the suction device 107 on the conveyor belt 108 and a fiber aggregate 109, in which a plurality of fibers 13 layer, is formed. The fiber aggregate 109 is continuous in the direction in which the fibers 13 are conveyed (the direction indicated by an arrow 209) and the direction in which the fiber aggregate 109 is continuous (the direction indicated by arrow 209) becomes the longitudinal direction of the interlabial pad 10.

Here, in the process of layering the fibrillated fibers 13 above the conveyor belt 108, the speed of capturing the fibers 13 is made greater than the conveying speed of the conveyor belt 108. When the speed of capturing the fibers 13 is greater than the conveying speed of the conveyor belt 108, the fibers 13 will be unlikely to be oriented in the direction in which the fibers 13 are conveyed, and the fiber orientations of the fibers 13 that form the fiber aggregate 109 can be made random. The speed of capturing fibrillated fibers 13 is mainly determined by the suction force of the suction device 107. When the suction pressure is, for example, in the range from 1500 to 15000Pa, the capture speed for capturing fibrillated fibers 13 is between 4 and 200 m/sec (240 to 1200 m/min).

As mentioned above, the conveying speed of the conveyor belt 108 is preferably set to between 20 and 200m/min, and here in order to make the speed of capturing the fibers 13 greater, the suction pressure is preferably set in the range from 1500 to 15000Pa. When the suction pressure is less than 1500Pa, the conveying speed will be greater than the capturing speed and the fibers 13 will orient readily in the longitudinal direction of the interlabial pad 10. On the other hand, when the suction pressure is more than 15000Pa, the fibers 13 become excessively entangled with the mesh of the conveyor belt 108 and it becomes difficult to transfer the fiber aggregate 109 to subsequent processes.

The fiber aggregate 109, formed by layering fibrillated fibers 13, is not drawn intentionally but is conveyed in a state in which the fiber orientations are maintained as they are when the fibers 13 were layered. A flexibility of the fiber aggregate 109 is then controlled by passing the fiber aggregate 109 between a pair of emboss rollers 110 that rotate in the direction indicated by an arrow 210. It is not preferable to intentionally draw the fiber aggregate 109 prior to embossing since the fibers 13, which form fiber aggregate 109, move too freely and the fiber orientations thus tend to be directed readily in the longitudinal direction of the interlabial pad 10.

The rotation speed of the emboss rollers 110 is preferably set so that the ratio of the speed by which the fiber aggregate 109 passes across the surfaces of the emboss rollers 110 (referred to hereinafter as the "surface speed") to the conveying speed of the conveyor belt 108 will be in the range from 0.9 to 1.2 and more preferably in a range from 1.0 to 1.1. When the surface speed becomes excessively greater than the conveying speed, the fiber aggregate 109 becomes drawn and the fibers 13 will tend to be oriented readily in the longitudinal direction of the interlabial pad 10.

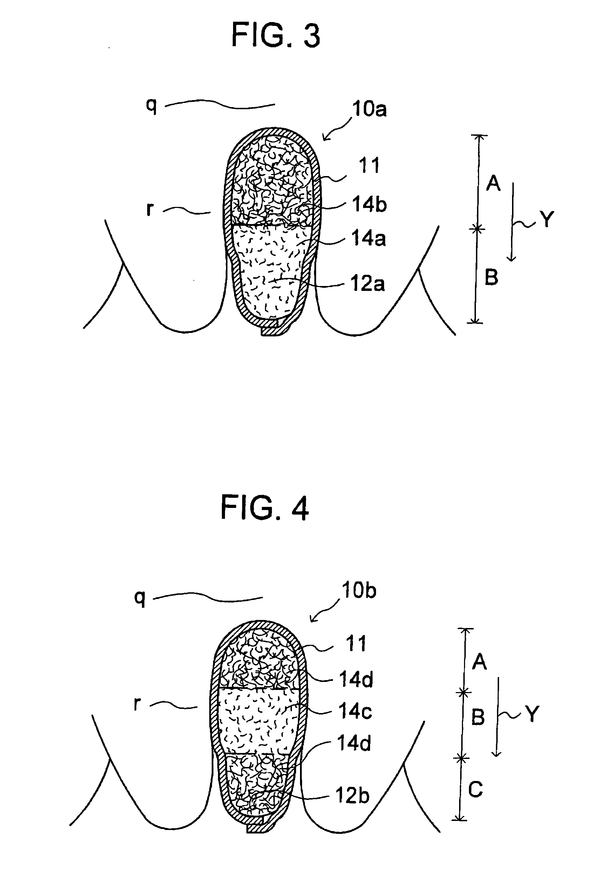

An interlabial pad 10a of a second embodiment of the present invention shall be described with reference to Fig. 3. Fig. 3 is a diagram showing the state in which the interlabial pad 10a of the second embodiment is fitted between labia r.

The absorbent body 12a, which makes up the interlabial pad 10a, has two types of fiber aggregates that differ in tensile elongation in the thickness direction (direction indicated by an arrow Y) of the interlabial pad 10a. Specifically, a side of vestibular floor q (referred to hereinafter as the "vestibular floor side") that becomes an upper region A and is positioned at a head side when the interlabial pad 10a is fitted on, is formed of a high tensile elongation fiber aggregate 14b comprising the fibers having a long fiber length an. Meanwhile, a lower region B, which is positioned at the side opposite to the vestibular floor q and at the lower limb side when the interlabial pad 10a is fitted on (this side shall be referred to hereinafter as the "side opposite to the vestibular floor side"), is formed of a low tensile elongation fiber aggregate 14a comprising the fibers having a short fiber length. The high tensile elongation fiber aggregate 14b has a higher tensile elongation than that of the low tensile elongation fiber aggregate 14a.

The high tensile elongation fiber aggregate 14b is formed by layering solitary or mixed fibers of rayon, acetate, natural cotton, super absorbent polymer fibers, synthetic fibers, etc. and has a maximum tensile elongation between 15 and 130% and preferably between 15 and 60%. It is not preferable that the maximum tensile elongation is less than 15%, since the spatial shapes between fibers will not deform readily and this may cause a wearer to feel a foreign-object sensation. Meanwhile, a maximum fiber elongation is not preferable to be more than 130%, since the shapes between fibers will deform more than necessary, causing absorbent body 12a to become lopsided and differ in bulk at parts and thus causing a foreign-object sensation to be felt. The average fiber length of the fibers that form the high tensile elongation fiber aggregate 14b is in the range from 10 to 501 mm and more preferably from 25 to 50 mm.

The low tensile elongation fiber aggregate 14a is formed by layering solitary or mixed fibers of pulp, chemical pulp, rayon, acetate, natural cotton, super absorbent polymer fibers, synthetic fibers, etc. and has a maximum tensile elongation not exceeding 10% and more preferably not exceeding 8%. A maximum tensile elongation is not preferable to exceed 10%, since the spatial shapes between fibers will deform readily and body fluids held between fibers will leak out readily. The average fiber length of the fibers that form the low tensile elongation fiber aggregate 14a is no more than 8 mm and is more preferably in the range from 3 to 6 mm.

Also, in order to provide good following properties not just with respect to the vestibular floor but with respect to the inner labial walls as well, the maximum tensile elongation of the absorbent body 12a as a whole is preferably set in the range from 10 to 130% and more preferably in the range from 15 to 60%. The maximum tensile strength of the absorbent body 12a is in the range from preferably 100 to 1500cN/25mm, more preferably from 200 to 1000cN/25mm, and most preferably from 250 to 500cN/25mm.

The factors for controlling the maximum tensile elongation of the absorbent body 12a include the fiber length of the fibers that make up the fiber aggregate, crimp rate of the fibers, surface friction of the fibers, and the method of forming the absorbent body 12a. Thus the high tensile elongation fiber aggregate 14b is preferably formed from solitary or mixed fibers having a crimp rate of the range from 20 to 80% with respect to total fiber length and a surfactant attached to the fibers at an amount in the range from 0.05 to 0.5%. When a fiber aggregate formed of crimped fibers is used, since the fibers stretch when the vestibular floor stretches and the fibers return to the original crimped shapes when the vestibular floor returns to the original state, the shape of the interlabial pad can be deformed repeatedly to match the movements of the wearer and good close contact can be realized.

The fibers which form the high tensile elongation fiber aggregate 14b positioned at the upper region A readily become entangled with each other, since the average fiber length is long. Thus when the entirety of the absorbent body 12a is stretched, the spaces between fibers deform in the high tensile elongation fiber aggregate 14b in a manner such that the fibers entangled become disentangled and consequently, the maximum tensile elongation of the absorbent body 12a as a whole is high. When the maximum tensile elongation is high, even when the vestibular floor stretches changes shape, the high tensile elongation fiber aggregate 14b will follow the change without undergoing separation and thus make it unlikely for a wearer to feel a foreign-object sensation.

Meanwhile, the fibers which forms the low tensile elongation fiber aggregate 14a positioned at lower region B, do not readily become entangled with each other and the coupling between fibers is low, since the average fiber length is short. Thus when the entirety of the absorbent body 12a is stretched, the fibers separate from each other readily at parts at which the fiber entanglement is weakest before the spaces between fibers deform and the maximum tensile elongation is thus low. When the maximum tensile elongation is low, though there is the possibility that the low tensile elongation fiber aggregate 14a will separate in response to a movement in which the vestibular floor stretches, since the spaces between fibers do not deform, body fluids that are held between fibers can be held without leakage.

The absorbent body 12a can thus deform in accordance with the body at the upper region A, which contacts the vestibular floor q, and can hold body fluids at the lower region B, which is separated from the body. Also, even when the low tensile elongation fiber aggregate 14a at the lower region B separates and becomes overlapped, the absorbent body 12 is unlikely irritate the vestibular floor q, since the high tensile elongation fiber aggregate 14b is in contact with vestibular floor q.

A third embodiment of the present invention shall now be described with reference to Fig. 4. Fig. 4 is a diagram showing the state in which an interlabial pad 10b of the third embodiment is fitted between labia. An absorbent body 12b, which makes up the interlabial pad 10b, is formed of two high tensile elongation fiber aggregates 14d, and a low tensile elongation fiber aggregate 14c disposed between the two high tensile elongation fiber aggregates 14d. The high tensile elongation fiber aggregates 14d and the low tensile elongation fiber aggregate 14c are layered in a direction parallel to the thickness direction Y, with the high tensile elongation fiber aggregates 14d being disposed at an upper region A closest to the vestibular floor q and a lowest region C farthest from the vestibular floor q, and the low tensile elongation fiber aggregate 14c having a short fiber length being disposed at a lower region B positioned between above-mentioned two high tensile elongation fiber aggregates 14d.

Since the lowest region C tends to protrude out from the labia and readily receives external impacts due to a napkin or something else that is used in combination, the interlabial pad 10b falls off readily. However, the external impacts can be cushioned and the interlabial pad 10b can be prevented from falling off by positioning the high tensile elongation fiber aggregate 14b having a high flexibility of fibers at lowermost region C.

The upper region A, the lower region B, and the lowest region C shall now be described.

Though the depth of labia differs according to each individual, the average value is approximately 14 mm. Thus parts of an interlabial pad that are positioned lower than 14 mm from the vestibular floor tends to protrude out from the labia when the interlabial pad is fitted between labia. That is, in the case of the interlabial pad 10b shown in Fig. 4, the region positioned below 14 mm from the uppermost part (the part in contact with the vestibular floor) of the upper region A tends to protrude out readily from the labia, and this region is referred to as the lowest region C. Meanwhile, the upper region A refers to the region, which extends 7 mm downwards from the position in contact with the vestibular floor in a state in which the interlabial pad is fitted the labia, and the lower region B refers to the region extending 7 mm further downwards.

The labia have an average length from the ventral side to the dorsal side of approximately 55 mm and extend by 50 mm from the ostium vaginae to the ventral side and by 5 mm to the dorsal side. Thus at a region extending outwards beyond 50 mm towards the ventral side from the position at which the interlabial pad contacts the ostium vaginae and at a region extending outwards beyond 5 mm towards the dorsal side, the interlabial pad tends to protrude out from the labia in the longitudinal direction.

Though the divisions of the upper region A, the lower region B, and the lowest region C were described above, these are not defined by the form of an interlabial pad prior to being fitted between the labia but are defined in the state in which the interlabial pad is fitted between the labia.

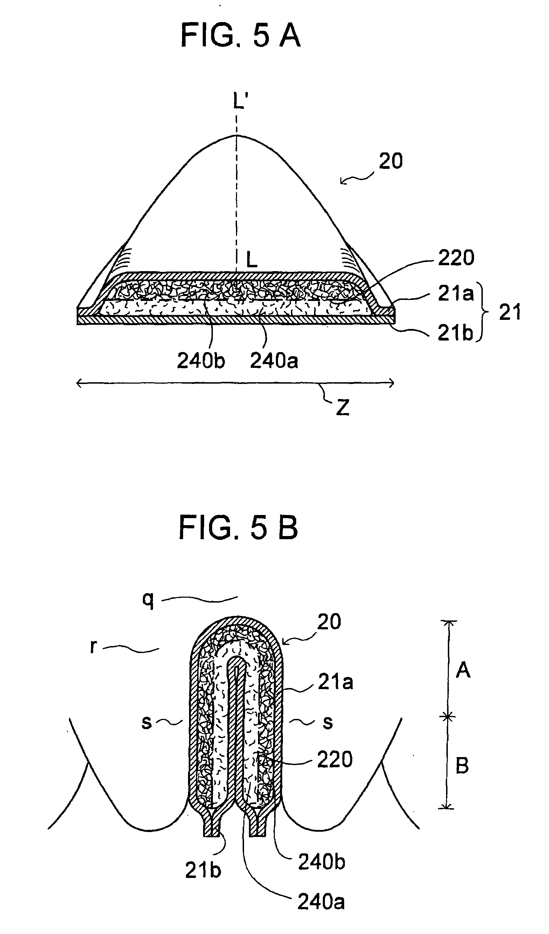

A fourth embodiment of the present invention shall now be described with reference to Fig. 5A and Fig. 5B. Fig. 5A is a perspective sectional view of an interlabial pad 20 of the fourth embodiment and Fig. 5B is a diagram showing the state in which the interlabial pad 20 is fitted between labia.

As shown in Fig. 5A, the interlabial pad 20 has a cover body 21 comprising a liquid permeable surface side sheet 21 a and a liquid impermeable back face sheet 21b, and an absorbent body 220 enclosed in this cover body 21. The interlabial pad 20 has a planar form of substantially elliptical shape in plan view. The absorbent body 220 is formed by a low tensile elongation fiber aggregate 240a and a high tensile elongation fiber aggregate 240b being uniformly layered in the thickness direction. The low tensile elongation fiber aggregate 240a is positioned at a side contacted to the back face sheet 21 b and the high tensile elongation fiber aggregate 240b is positioned at a side contacted to the surface side sheet 21 a.

As shown in Fig. 5B, the interlabial pad 20 has a longitudinal direction central line L-L' as a symmetry axis and is fitted between labia being folded so that the back face sheet 21b faces each other. The high tensile elongation fiber aggregate 240b having a high flexibility among fibers contacts not just the vestibular floor q but also the inner labial walls s and the interlabial pad 20 can follow the change of the inner labial walls s even better.

Since the low tensile elongation fiber aggregate 240a and the high tensile elongation fiber aggregate 240b are layered substantially uniformly, the weight proportions of the low tensile elongation fiber aggregate 240a and the high tensile elongation fiber aggregate 240b are substantially the same at an upper region A and a lower region B. The tensile elongation of the upper region A as a whole is thus substantially the same as the tensile elongation of the lower region B as a whole. Also, since the high tensile elongation fiber aggregate 240b having the high flexibility of fibers is positioned at the surface side of the upper region A that contacts the vestibular floor q, the interlabial pad 20 can follow the stretching change of the vestibular floor q.

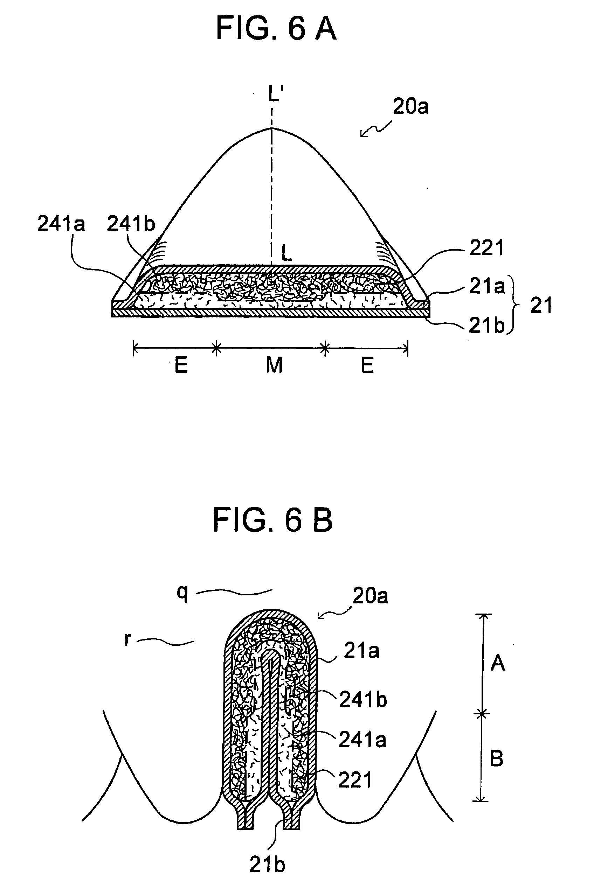

A fifth embodiment of the present invention shall now be described with reference to Fig. 6A and Fig. 6B. Fig. 6A is a perspective sectional view of an interlabial pad 20a of the fifth embodiment and Fig. 6B is a diagram showing the state in which the interlabial pad 20a is fitted between labia.

An absorbent body 221 of the interlabial pad 20a has a low tensile elongation fiber aggregate 241a and a high tensile elongation fiber aggregate 241b, and both low tensile fiber aggregate 241a and high tensile elongation fiber aggregate 241 b vary in fiber amount along the lateral direction. Specifically, the fiber amount at a central region M near the longitudinal direction central line L-L' is less than the fiber amount at a side regions E near the side parts in the lateral direction, and the thickness direction cross section is indented at the central part with regard to the low tensile elongation fiber aggregate-241a which is positioned at a side facing to the back face sheet 21b. Meanwhile, the fiber amount at the central region M is greater than the fiber amount at side regions E, and the thickness direction cross section protrudes at the central part with regard to the high tensile elongation fiber aggregate 241 b which is positioned at the surface side sheet 21 a side.

The ratio of the fiber amount at the central region M and the fiber amount at the side part regions E of low tensile elongation fiber aggregate 241a is preferably in the range from 0 to 40 weight %: from 100 to 60 weight %. In view of making body fluids be transferred smoothly from the high tensile elongation fiber aggregate 241 b to the low tensile elongation fiber aggregate 241a, the range from 10 to 30 weight % : from 90 to 70 weight % is more preferable. Also, the central region M having less amount of fiber has a width of 5 to 40% and preferably 20 to 30% of the width of the absorbent body 23.

For the low tensile elongation fiber aggregate 241a, a fiber aggregate having the same material and fiber length as the low tensile elongation fiber aggregate 14a, used in the absorbent body 12a of the interlabial pad 10a shown in Fig. 3 may be used. Likewise, for the high tensile elongation fiber aggregate 241b, a fiber aggregate having the same material and fiber length as the high tensile elongation fiber aggregate 14b used in the absorbent body 12a of the interlabial pad 10a may be used.

As shown in Fig. 68, the weight proportions of the low tensile elongation fiber aggregate 241 a and the high tensile elongation fiber aggregate 241 b differ at the upper region A which is positioned close to the vestibular floor q and at the lower region B which is positioned further below, when the interlabial pad is fitted between labia. Thus, the tensile elongation of the upper region A, in which the proportion of the high tensile elongation fiber aggregate 241 is high, will be greater than the tensile elongation of the lower region B. Therefore, even if an external pressure that pushes upwards is applied when a wearer moves like sitting down a chair, etc., the stiff sensation caused by the low tensile elongation fiber aggregate 241a can be cushioned by the high tensile elongation fiber aggregate 241b so that the vestibular floor q will not be irritated readily and the wearer can be prevented from feeling a foreign-object sensation.

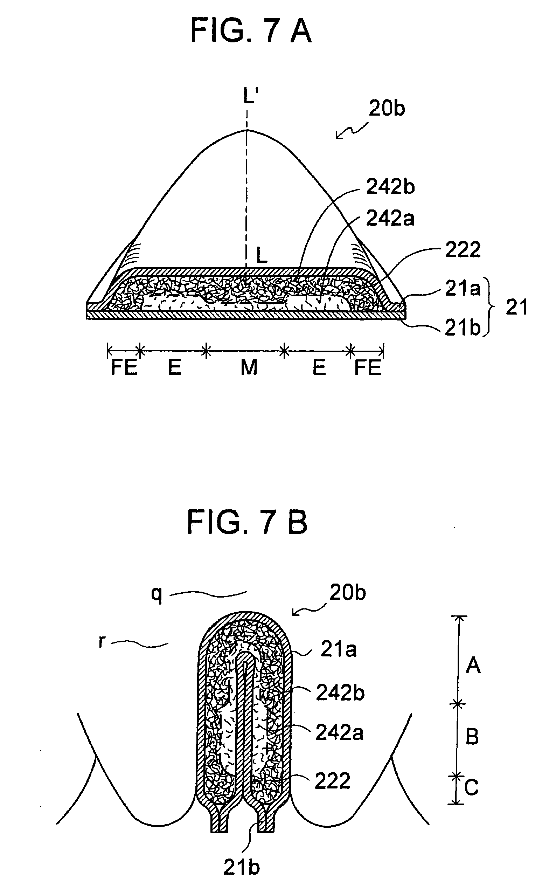

A sixth embodiment of the present invention shall be described. Fig. 7A is a perspective sectional view of an interlabial pad 20b of the sixth embodiment and Fig. 7B is a diagram showing the state in which the interlabial pad 20b is fitted between labia.

A low tensile elongation fiber aggregate 242a and a high tensile elongation fiber aggregate 242b which make up the interlabial pad 20b are both formed so as to vary in fiber amount in the lateral direction. The same component material as that used for the low tensile elongation fiber aggregate 241a shown in Fig. 6A may be used as the component material of the low tensile elongation fiber aggregate 242a. Likewise, the same component material as that used for the high tensile elongation fiber aggregate 241 b of Fig. 6A may be used as the component material of the high tensile elongation fiber aggregate 242b.

As shown in Fig. 7A, the low tensile elongation fiber aggregate 242a which is positioned at the back face sheet 21 b side is formed so that the fiber amount at a central region M will be less than the fiber amount at the side regions E positioned at both sides of the central region M. Meanwhile, the high tensile elongation fiber aggregate 242b which is positioned at the surface side sheet 21a side is formed so that the fiber amount increases in the order of: the side regions E; the central region M; and a far end regions FE which are adjacent outer edges in the lateral direction of the interlabial pad 20.

Thus when this interlabial pad 20b is fitted between labia as shown in Fig. 7B, the high tensile elongation fiber aggregate 242b is positioned at the lowest region C. Thus, a foreign-object sensation that arises due to the lowest region C protruding from the labia and contacting the underwear, etc. can be reduced when the interlabial pad 20b is worn. For an interlabial pad like the interlabial pad 20b which changes the form being folded in two when its is fitted between labia, the lowest region C refers to the regions extending more than 14 mm each in the outer edge direction from a longitudinal central line L-L'.

The low tensile elongation fiber aggregate 242a is, for example, formed by fibrillating a pulp having a fiber length from 1 to 8 mm and a fiber diameter in the range from 10 μm to 60 μm by the air laid method, and layering the fibers at a specific weight of 10g/m$^2$ in the direction of the respective outer edges from the longitudinal direction central line L-L' to positions up to 7 mm, and then layering the fibers at a specific weight of 100g/m$^2$ in the direction of the respective outer edges from the longitudinal direction central line L-L' to positions up to 20 mm. The lateral width (width of the central region M + width of each side region E $\times$ 2) of the low tensile fiber aggregate 242a will thus be 40 mm. Meanwhile, the high tensile elongation fiber aggregate 242b is, for example, formed by fibrillating rayon fibers having a fiber length of 51 m, a crimp rate of fiber of 50%, and a fineness of 3.3dtex and a hydrophilic oil attached at an amount of 0.2% by the air laid method, and layering the fibers thus obtained onto the indented surface of the above-described low tensile elongation fiber aggregate 242a at a specific weight of 180g/m$^2$ at positions up to approximately 30 mm in the direction of the respective outer edges from longitudinal central line L-L'.

The low tensile elongation fiber aggregate 242a and the high tensile elongation fiber aggregate 242b are layered and embossed to have an emboss percentage of 1.8% in a state of being layered and cut to form the absorbent body 222. The absorbent body 222 is positioned between the surface side sheet 21 a and the back face sheet 21 b, and the surface side sheet 21a and the back face sheet 21b are cut at positions 2 mm to 5 mm to the outer side of the outer edge parts of the absorbent body 222 and joined each other to form the interlabial pad 20b. These procedures are given as an example and the present invention is not limited thereto.

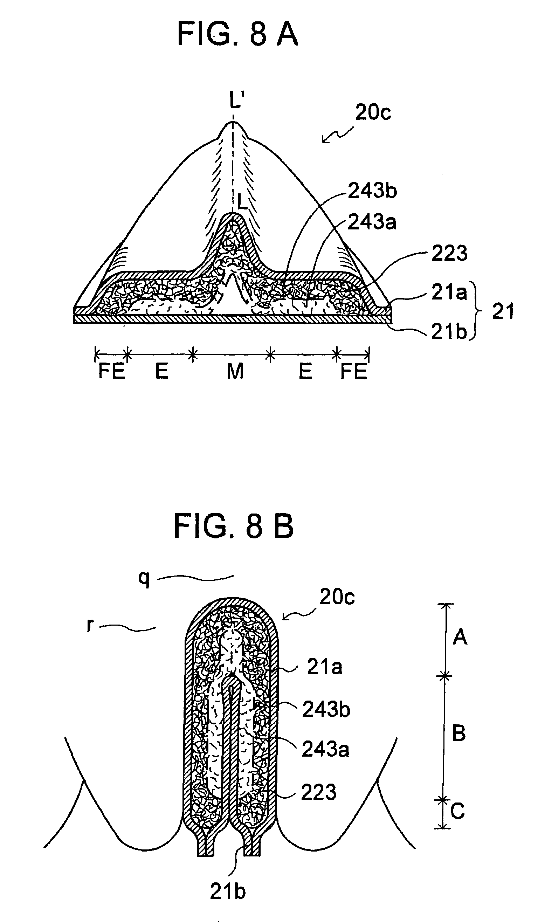

A seventh embodiment of the present invention shall now be described with reference to Fig. 8A and Fig. 8B. Fig. 8A is a perspective sectional view of an interlabial pad 20c of the seventh embodiment and Fig. 8B is a diagram showing a state in which the interlabial pad 20c is fitted between labia.

The absorbent body 223 of the interlabial pad 20c has a projected part which extends from the longitudinal direction central line L-L' to the points approximately 7 mm from the central line L-L' in the directions of the respective outer edges (the part positioned at central region M) and protruded towards the surface side sheet 21a side. The component materials of a high tensile elongation fiber aggregate 243b and a low tensile elongation fiber aggregate 243a may be made the same as those of the high tensile elongation fiber aggregate 242b or the low tensile elongation fiber aggregate 242a that form the absorbent body 222 of Fig. 7A.

With the present embodiment, since the absorbent body 223 protrudes towards the surface side sheet 21a side near the central region M, a wearer can hold the longitudinal direction central line L-L' of the interlabial pad 20c definitely. Fitting in a manner that is symmetrical in the left/right directions can be performed readily in the fitting process to achieve good close contact of the interlabial pad 20c with the inner labial walls. The absorbent body positioned at the central region M of the absorbent body 220 of interlabial pad 20 shown in Fig. 5A or the interlabial pad 20a shown in Fig. 6A may also be protruded towards the surface side sheet 21a side.

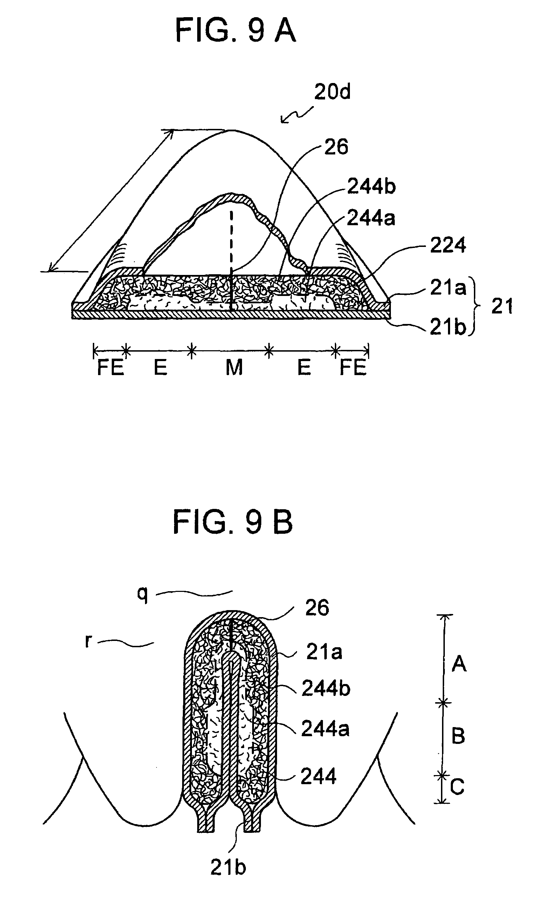

An eight embodiment of the present invention shall now be described with reference to Fig. 9A and Fig. 9B. Fig. 9A is a perspective sectional view of an interlabial pad 20d of the eighth embodiment of the present invention. Fig. 9B is a diagram showing the state in which the interlabial pad 20d of the eighth embodiment of the present invention is fitted between labia.

The interlabial pad 20d of the eighth embodiment has an absorbent body 224, wherein perforation-like slits 26 are formed along a longitudinal direction central line L-L' and from the center to the rear end in the longitudinal direction (the end part at the side positioned at the dorsal side when the interlabial pad 20d is fitted on) as shown in Fig. 9A. These slits 26 are formed through the entirety of the thickness direction of the absorbent body 224 in a perforation-like slit pattern, with which the slit length is 5 mm to 20 mm and the slit pitch is 5 mm to 20 mm. By making the length of each slit 26 longer, the repulsive force that arises when the interlabial pad 20 is bent can be made less likely to act. Thus the longer the length of each slit 26, the more preferable.

In comparison to the high tensile elongation fiber aggregate 244b, the low tensile elongation fiber aggregate 244a, which is formed of fibers having a short fiber length, is more readily increased in density in the wetted state in which body fluids are absorbed and is also more readily increased in repulsive force when bent. The slits 26 are thus preferably formed so as to divide at least the low tensile elongation fiber aggregate 244a at the back face sheet 21 b side, and as in the present embodiment, may be cut across the entirety in the thickness direction so as to divide not just the low tensile elongation fiber aggregate 244a but also the high tensile elongation fiber aggregate 244b as well.

Furthermore, the repulsive force of a region of the interlabial pad 20d which protrudes to the dorsal side of the labia, that is a region positioned so as to contact the perineum and the anus is preferably substantially the same as the repulsive force of the part in contact with the labia at the dorsal side. When the repulsive force of the part positioned at the region that protrudes to the dorsal side of the labia is high, the fat and muscle in the vicinity of the anus may be irritated more than necessary and a wearer may feel a foreign-object sensation. The slits 26 are thus preferably provided so as to reach up to the rear end in the longitudinal direction of the absorbent body 224.

As the specific position at which the slits 26 are to be provided, the region of the interlabial pad 20d extending to the dorsal side from the part, which is in contact with the vestibular floor or the inner labial walls and is located approximately 25 mm to the ventral side of the ostium vaginae, is preferable.

The slits may also be formed not just in the absorbent body 224 but in the surface side sheet 21 a as well. Since the absorbent body 224 is enclosed between the surface side sheet 21a and the back face sheet 21b and is furthermore fitted upon being folded, the surface side sheet 21a becomes taught and a repulsive force against bending that tends to open up the labia acts. On the other hand, if slits are provided in the surface side sheet 21a, since the slit parts open up upon folding, the repulsive force against bending will be less likely to act and the vicinity of the ostium vaginae will less likely to become irritated.

Also, slits may be provided not just at a rear part of the absorbent body 224 (in the direction towards the dorsal side in the fitted state) but also at a front part thereof (in the direction towards the ventral side in the fitted state). Since a force holding an interlabial pad caused labia is high at the ventral side of the labia, the interlabial pad 20d is more readily prevented from falling off if a repulsive force against bending of a degree that will not make a wearer feel a foreign-object sensation is provided. Thus when slits are provided at a front part of absorbent body 224, the length of each of these slits is preferably made shorter than the length of each slit 26 made at a rear part of absorbent body 224.

Slits may also be provided in the interlabial pad 20 shown in Fig. 5A, or in the interlabial pad 20a shown in Fig. 6A.

Also as another method for increasing the tensile elongation of the absorbent body, slits that extend in the direction orthogonal to the direction in which the elongation is to be increased may be provided in the region which is to be increased in tensile elongation. When the absorbent body 224 is thus divided by providing slits, since the divided parts open up in response to movements of the body, the tensile elongation increases, and at the same time, the tensile strength can be decreased to enable a flexibility of whole of the absorbent body 224 to increase.

Also, the elongation can be controlled by means of the pitch of the slit pattern in the above-described slitting process, and the elongation can be increased by narrowing the pitch. That is, though slitting may be applied to the entirety of the absorbent body 224, the slit pattern is preferably such that the pitch at an upper region or a lowest region is set narrower than the pitch at a lower region.

Furthermore, when the slits 26 are provided at the lowest region C so as to reach outer edge parts of the absorbent body 224, the absorbent body 224 is divided so as to open up in the direction of the outer edges, thus enabling better cushioning of external impacts and making it less likely for a foreign-object sensation to be felt even when the fitting point of an interlabial pad is searched for.

The MD/CD ratio of flexural rigidity can also be controlled by means of the direction of the slit pattern, and it is preferable that the pitch towards MD and the pitch towards CD are substantially equal.

(Example 1)

The interlabial pad of the eighth embodiment shown in Fig. 9A is used as Example 1. The raw material of the absorbent body of the interlabial pad is rayon having a fiber length of 51 mm, a fiber length crimp rate of 50%, a fineness of 3.3dtex, and a hydrophilic oil attached at an amount of 0.2%. The abovementioned rayon was subject to be fibrillated by the air laid method using the garnet type fiber fibrillating method and layered into a fiber aggregate at a high suction force to maintain a random fiber orientation. After being conveyed so that tension will not be applied excessively during the conveying process, the fiber aggregate was embossed to an emboss percentage of 1.8% using a dotted emboss pattern to form an absorbent body with a specific weight of 304g/m$^2$.

The conditions for forming the absorbent body were: a suction pressure of 4000Pa; a conveying speed of 80m/min; and a ratio of the circumferential velocity of the emboss roller to the circumferential velocity of the conveyor belt of 1.2. The dot-form emboss roller had dot having a diameter of 1.5 mm aligned in zigzag form at a pitch of 12 mm.

The absorbent body had a maximum tensile elongation between 22 and 50%, a maximum tensile strength between 498 and 689CN/25mm, an apparent density of 0.055g/cm$^3$, and a ratio of the longitudinal direction flexural rigidity to the lateral direction flexural rigidity (MD/CD ratio of flexural rigidity) of 1.1.

(Example 2)

As Example 2, besides embossing to an emboss percentage of 25% using an emboss pattern of dot having a diameter of 2.4 mm, an interlabial pad of the same arrangement as Example 1, is used.

(Comparative Example 1)

As Comparative Example 1, the interlabial pad of the trade name, "Envive," made by PG Corp. is used.

(Comparative Example 2)

As Comparative Example 2, the interlabial pad of the trade name, "INSYNC," made by A-Fem Medical Corporation is used.

(Comparative Example 3)

An interlabial pad used for Comparative Example 3 is the same as the interlabial pad of Example 1 except an absorbent body. The absorbent body of the interlabial pad of Comparative Example 3 is formed of pulp having a fiber length of 1 to 8 mm and has a MD/CD ratio of flexural rigidity (that is a ratio of the flexural rigidities of the mutually orthogonal directions) of 2.4.

The arrangement and the physical properties of total specific weight, total density, and flexural rigidity and tensile characteristics in the dry state of the absorbent body and the results of an actual usage test are shown for the absorbent bodies of the interlabial pads of the above-described Examples and the above-described Comparative Examples in Table 1 and Table 2. The method of measuring the flexural rigidity shall be described later. The actual usage test was carried out with 20 women on the first to third day of menstruation and by using 5 panels each, setting the duration of fitting between the labia to 2 to 3 hours, and checking the "foreign-object sensation" and "number of pads that fell off" after use. In fitting on an interlabial pad, the napkin of the trade name, "Sofy Sara hin" (registered trademark), made by UniCharm Corp. was fitted on in combination.

[Table 1]

| | | | Example1 | Example2 | Comparative Example1 | Comparative Example2 | Comparative Example3 |
|---|---|---|---|---|---|---|---|
| Arrangement of the absorbent body | Surface side cover member | Material | — | — | — | — | — |
| | | Specific weight(g/m²) | — | — | — | — | — |
| | Absorbent layer | Material | Web-rayon 100% (3.3dl×51mm) | Web-rayon 100% (3.3dl×51mm) | Fiber aggregate (Cellulose) | Fiber aggregate (Cellulose) | Pulp |
| | | Specific weight(g/m²) | 304 | 304 | 360 | 580 | 295 |
| | Backside cover member | Material | — | — | — | — | — |
| | | Specific weight(g/m²) | — | — | — | — | — |
| Secondary elaboration | Embossing | | Dotted emboss percentage: 1.8% | Dotted emboss percentage: 25% | Dotted emboss percentage: 0.5% | — — | Dotted emboss percentage: 1.8% |
| Total specific weight of the absorbent body(g/m²) | | | 304 | 304 | 360 | 580 | 295 |
| Total density of the absorbent body(g/cm³) | | | 0.055 | 0.071 | 0.048 | 0.097 | 0.070 |

EP 1 598 038 A1

[Table 2]

| | | | Example1 | Example2 | Comparative Example1 | Comparative Example2 | Comparative Example3 |
|---|---|---|---|---|---|---|---|
| Flexural rigidity (as Gurley bending) in dry state | MD direction(mg) | | 29 | 128 | 33 | 368 | 21 |
| | CD direction (mg) | | 27 | 110 | 14 | 124 | 19 |
| | MD/CD*1 | | 1.1 | 1.2 | 2.4 | 3.0 | 1.1 |
| Tensile characteristics in dry state | MD direction | Maximum strength(cN/25mm) | 689 | 1068 | 4033 | 28035 | 16 |
| | | Maximum elongation(%) | 22 | 15 | 15 | 28 | 9 |
| | CD direction | Maximum strength(cN/25mm) | 498 | 867 | 83 | 19 | 15 |
| | | Maximum elongation(%) | 50 | 38 | 25 | 131 | 12 |
| | MD/CD*2 | Maximum strength | 1.4 | 1.2 | 48.4 | 1475.5 | 1.1 |
| Actual usage test[3] | Percentage of women feeling foreing-object sensation (%) | | 15 | – | 15 | 60 | 25 |
| | Percentage of pads fallen off during usage(%) | | 21 | – | 29 | 21 | 38 |

1) MD/CD*1: The flexural rigidity (Gurley bending) in the MD direction／The flexural rigidity (Gurley bending) in the CD direction

2) MD/CD*2: The ratio of the maximum tensile elongation in the MD direction／The maximum tensile elongation in the CD direction

3) Actual usage test*3: 20 panelists use 5 panels each.

The MD/CD ratios of flexural rigidity of the absorbent bodies of the interlabial pads of Comparative Example

15

1 and Comparative Example 2 were extremely high, since the fibers that form the absorbent bodies are mainly oriented in the lateral direction and have high fiber rigidity.

The absorbent body of the interlabial pad of Comparative Example 1 is extremely high in bendability having the flexural rigidities of 33 mg in the longitudinal direction and of 14 mg in the lateral direction, is also high in flexibility among fibers, and furthermore has a high MD/CD ratio of flexural rigidity of 2.4. This is due to setting the apparent density of the absorbent body to a low value of 0.048g/cm$^3$ and thereby providing a large number of spaces between fibers and increasing the flexibility among fibers. However, since the MD/CD ratio of flexural rigidity of the absorbent body is made high and the flexibility is made too high, the absorbent body became lopsided readily in the lateral direction while the interlabial pad was fitted on, causing the are contacting with the inner labial walls to decrease and also causing the percentage of fall-off of the interlabial pad to be high.

Meanwhile, the absorbent body of the interlabial pad of Comparative Example 2 is high in flexural rigidities being 368 mg in the longitudinal direction and 124 mg in the lateral direction, and also has a high MD/CD ratio of flexural rigidity of 3.0. However, the absorbent body has a large apparent density of 0.097g/cm$^3$ and is restrained in the flexibility among fibers. Though the absorbent body was thus less likely to become lopsided in the lateral direction during fitting, the proportion of occurrence of complaints of a foreign-object sensation was high.

Also, with the absorbent body of Comparative Example 3, though the flexural rigidity can be made low since both the tensile elongation and tensile strength are low, separation of the absorbent body also occurs readily. Thus not only was the percentage of fall-off high but the proportion of occurrence of complaints of a foreign-object sensation was also high.

These show that in order to prevent a wearer from feeling a foreign-object sensation and prevent the absorbent body from becoming lopsided during fitting and the fall-off percentage from becoming high, the MD/CD ratio of flexural rigidity of an interlabial pad must be controlled to be in the range from 0.5 to 2.0 and the flexural rigidity of the interlabial pad must be controlled to be in the range from 25 to 130 mg and more preferably in the range from 35 to 90 mg.

It was also shown that in order to control these flexural rigidities, the maximum tensile elongation should be in the range from 10 to 130% and more preferably from 15 to 60%, the apparent density of the absorbent body should be in the range from 0.050 to 0.085g/cm$^3$ and more preferably from 0.055 to 0.075g/cm$^3$, the maximum tensile strength of the absorbent body should be in the range from 100 to 1500cN/25mm and more preferably from 200 to 1000cN/25mm, and the MD/CD ratio of maximum tensile strength should be in the range from 0.5 to 2.0.

Also, though the absorbent body of the interlabial pad of Example 2 is substantially the same in arrangement and preparation conditions as the absorbent body of the interlabial pad of Example 1, it has the emboss pattern provided dot having the diameter of 2.4 mm and the emboss percentage of 25%. It was thus demonstrated as shown in Table 1 that the flexibility among fibers can be controlled and the MD/CD ratio of flexural rigidity can also be controlled by the emboss pattern and the emboss percentage.

The emboss percentage is thus set in the range from 0.6 to 30% and preferably from 1.0 to 10%, and though not restricted, the emboss pattern is preferably not a continuous emboss pattern but is an intermittent pattern in consideration of the method of controlling the flexural rigidities of the absorbent body and controlling the MD/CD ratio of flexural rigidity. A dotted pattern can be cited as an example of an intermittent emboss pattern and furthermore it is preferable for the pitch of the dot forms to be such that the pitch in the longitudinal direction and the pitch in the lateral direction are substantially uniform.

The results of having 20 panelists actually fit on 5 panels each were best with Example 1.

(Example 3)

As Example 3, the interlabial pad of the second embodiment of the present invention, shown in Fig. 3, is used. The high tensile elongation fiber aggregate that forms the absorbent body of the interlabial pad uses rayon having a fiber length of 51 mm, a fiber crimp rate of 50%, a fineness of 3.3dtex, and a hydrophilic oil attached at an amount of 0.2% as the raw material and is formed by layering fibers fibrillated by the air laid method. The low tensile elongation fiber aggregate is formed by fibrillating fibers having fiber lengths in the range from 1 to 8 mm and fiber diameters in the range from 10 to 60 μm by the air laid method and layering these fibers.

The tensile characteristics, when the absorbent body of this interlabial pad is put in the wetted state by making it absorb artificial menstrual blood (absorption percentage: 400%), were measured for an upper region and a lower region, and the results and the absorbent body characteristics are shown in Table 3. The measurement methods for the apparent density, absorption percentage, etc. and the method of preparing the artificial menstrual blood shall be described later.

[Table 3]

| Arrangement of the absorbent body | | | Upper region | Lower region |
|---|---|---|---|---|
| | Absorbent layer | Material | Web-rayon 100% (3.3dl×51mm) | Pulp |
| | | Specific weight (g/m²) | 180 | 80 |
| Total specific weight of the absorbent body (g/m²) | | | 180 | 80 |
| Total density of the absorbent body (g/cm³) | | | 0.055 | 0.073 |
| Tensile characteristics | | | | |
| | In dry state | MD direction Maximum elongation (%) | 24 | 7 |
| | | CD direction Maximum elongation (%) | 43 | 5 |
| | In wet state (Absorption percentage 400%) | MD direction Maximum elongation (%) | 22 | 4 |
| | | CD direction Maximum elongation (%) | 41 | 3 |
| | Wet tensile/Dry tensile*¹ | MD direction Maximum elongation (%) | 91 | 57 |
| | | CD direction Maximum elongation (%) | 94 | 40 |

1)Wet tensile/Dry tensile*; The ratio of the maximum tensile elongation in wet state/The maximum tensile elongation in dry state

EP 1 598 038 A1

As shown in Table 3, the high tensile elongation fiber aggregate that is positioned at the upper region exhibited an elongation of 90% or more than that of the dry state, the distances between fibers did not shrink, and there was a flexibility among the fibers even in the wetted state.

[Example 4]

As Example 4, an interlabial pad of the same arrangement, except an absorbent body, as the interlabial pad of the fourth embodiment of the present invention, shown in Fig. 5A, is used. The absorbent body of the interlabial pad of Example 4 has a three-layer structure; an absorbing layer, a surface side cover material covering the surface side sheet side of the absorbing layer, and a back face side cover material covering the back face sheet side of the absorbing layer, As the surface side sheet of this interlabial pad, a dry forming spun lace prepared by layering rayon fibers having a fiber length of 44 mm and fineness of 1.4dtex at a specific weight of 30g/m$^2$ and then forming a sheet by hydro-entanglement is sued. Also, the absorbent body of the interlabial pad was prepared by layering rayon fibers of 3.3dl × 51 mm at a specific weight of 180g/m$^2$, pulp of fiber having a length of no more than 8 mm at a specific weight of 80g/m$^2$, and tissue at a specific weight of 15g/m$^2$ in that order by the air laid method and embossing to an emboss percentage of 1.8% using a dotted emboss pattern.

The back face sheet is a liquid impermeable film sheet formed by adjusting the thickness of a polyethylene resin to 20 μm. On the surface at one side of the back face sheet at which the absorbent body is positioned ("body face side"), a heat-sensitive adhesive agent having EVA or other synthetic rubber as the main component was applied in a continuous multiple spiral form at a weight of 3g/m$^2$ specific weight so as to cover the entire surface of the body face side of the back face sheet, and thereafter the abovementioned absorbent body was overlapped.

Furthermore, in order to make the fitting position of the interlabial pad more definite, a mini-sheet piece formed of a nonwoven fabric with a specific weight of 18g/m$^2$ was disposed across a length of 50 mm from the rear end part of the interlabial pad on the surface at the side opposite to the body face side surface of the back face sheet, and an opening enabling the insertion of a finger between the mini-sheet piece and the back face sheet was provided. Both side parts of the mini-sheet piece were adhered to the back face sheet using a heat sensitive adhesive agent having EVA or other synthetic rubber as the main component, so that the opening became 25 mm in width.

The interlabial pad was made to have dimensions of a maximum width of 58 mm and a maximum length of 91 mm by cutting the so-called flap part which is comprised of the surface side sheet, the back face sheet, and the mini-sheet piece at positions 3 mm from the peripheral parts of the absorbent body.

(Example 5)

An interlabial pad which is the same as the interlabial pad of the fifth embodiment shown in Fig. 6A except for an absorbent body is used as Example 5. The absorbent body of the interlabial pad of Example 5 has a three-layer structure which is the same as that of Example 4. The absorbent body of this interlabial pad was prepared by forming a part corresponding to the upper region when the interlabial pad is fitted on by layering rayon fibers of 3.3dl × 51 mm at a specific weight of 180g/m$^2$, pulp having a fiber length of no more than 8 mm at a specific weight of 20g/m$^2$, and tissue at a specific weight of 15g/m$^2$ in that order by the air laid method, forming a part corresponding to the lower region by layering rayon fibers of 3.3dl × 51 mm at a specific weight of 180g/m$^2$, pulp having a fiber length of no more than 8 mm at a specific weight of 100g/m$^2$, and tissue at a specific weight of 15g/m$^2$ in that order by the air laid method, and then embossing to an emboss percentage of 1.8% using a dotted emboss pattern. The arrangement of other parts was made the same as Example 4.

The arrangement, density, and flexural rigidity and tension characteristics in a dry state of the absorbent body and the results of an actual usage test are shown for Examples 1, 4, and 5 and Comparative Examples 1, 2, and 3 in Table 4,5,6 and 7. The method of measuring the flexural rigidity, apparent density, etc. shall be described later. The actual usage test was carried out with 20 women on the first to third day of menstruation and by using 5 panels each, setting the duration of fitting between the labia to 2 to 3 hours, and checking the "foreign-object sensation" and "number of pads that fell off" after use. In fitting on an interlabial pad, the napkin of the trade name, "Sofy Sara hin" (registered trademark), made by UniCharm Corp. was fitted on in combination.

[Table 4]

| | | | Example1 | Example4 | | Example5 | |
|---|---|---|---|---|---|---|---|
| | | | | Upper region | Lower region | Upper region | Lower region |
| Arrangement of the absorbent body | Surface side cover member | Material | — | Web-rayon 100% (3.3dl×51mm) | Web-rayon 100% (3.3dl×51mm) | Web-rayon 100% (3.3dl×51mm) | Web-rayon 100% (3.3dl×51mm) |
| | | Specific weight (g/m²) | — | 180 | 180 | 180 | 180 |
| | Absorbent layer | Material | Web-rayon 100% (3.3dl×51mm) | Web-rayon 100% (3.3dl×51mm) | Pulp | Pulp | Pulp |
| | | Specific weight (g/m²) | 304 | 80 | 80 | 20 | 100 |
| | Backside cover member | Material | — | Tissue | Tissue | Tissue | Tissue |
| | | Specific weight (g/m²) | — | 15 | 15 | 15 | 15 |
| Secondary elaboration | Embossing | | Dotted Emboss percentage: 1.8% | Dotted Emboss percentage: 1.8% | | Dotted Emboss percentage: 1.8% | |

EP 1 598 038 A1

[Table 5]

| | | | Example1 | Example4 | | Example5 | |
|---|---|---|---|---|---|---|---|
| | | | | Upper region | Lower region | Upper region | Lower region |
| Total specific weight of the absorbent body (g/m²) | | | 304 | 275 | 275 | 215 | 295 |
| Total density of the absorbent body (g/cm³) | | | 0.055 | 0.060 | 0.060 | 0.057 | 0.062 |
| Flexural rigidity (as Gurley bending) in dry state | MD direction (mg) | | 29 | 110 | 110 | 78 | 119 |
| | CD direction (mg) | | 27 | 95 | 95 | 68 | 96 |
| | MD/CD*1 | | 1.1 | 1.2 | 1.2 | 1.1 | 1.2 |
| Tensile characteristics in dry state | MD direction | Maximum strength (cN/25mm) | 689 | 891 | 891 | 765 | 926 |
| | | Maximum elongation (%) | 22 | 17 | 17 | 19 | 17 |
| | CD direction | Maximum strength (cN/25mm) | 498 | 507 | 507 | 438 | 566 |
| | | Maximum elongation (%) | 50 | 28 | 28 | 31 | 29 |
| | MD/CD*2 | Maximum strength— | 1.4 | 1.8 | 1.8 | 1.7 | 1.6 |
| Actual usage test*3 | Percentage of women feeling foreing-object sensation (%) | | 15 | 25 | | 15 | |
| | Percentage of pads fallen off during usage(%) | | 21 | 16 | | 14 | |
| | Percentage of leakage to a napkin (%) | | 53 | 38 | | 38 | |

EP 1 598 038 A1

[Table 6]

| | | | Comparative Example1 | Comparative Example2 | Comparative Example3 |
|---|---|---|---|---|---|
| Arrangement of the absorbent body | Surface side cover member | Material | — | — | — |
| | | Specific weight (g/m²) | — | — | — |
| | Absorbent layer | Material | Fiber aggregate (Cellulose) | Fiber aggregate (Cellulose) | Pulp |
| | | Specific weight (g/m²) | 360 | 580 | 295 |
| | Backside cover member | Material | — | — | — |
| | | Specific weight (g/m²) | — | — | — |
| Secondary elaboration | Embossing | | Dotted Emboss percentage: 0.5% | — | Dotted Emboss percentage: 1.8% |

[Table 7]

| | Comparative Example1 | Comparative Example2 | Comparative Example3 |
|---|---|---|---|
| Total specific weight of the absorbent body (g/m²) | 360 | 580 | 295 |
| Total density of the absorbent body (g/m³) | 0.048 | 0.097 | 0.070 |
| Flexural rigidity (as Gurley bending) in dry state — MD direction (mg) | 33 | 368 | 21 |
| CD direction (mg) | 14 | 124 | 19 |
| MD/CD*1 | 2.4 | 3.0 | 1.1 |
| Tensile characteristics in dry state — MD direction — Maximum strength (cN/25mm) | 4033 | 28035 | 16 |
| Maximum elongation (%) | 15 | 28 | 9 |
| CD direction — Maximum strength (cN/25mm) | 83 | 19 | 15 |
| Maximum elongation (%) | 25 | 19 | 12 |
| MD/CD*2 — Maximum strength— | 48.4 | 1475.5 | 1.1 |
| Actual usage test*3 — Percentage of women feeling foreing-object sensation (%) | 15 | 60 | 25 |
| Percentage of pads fallen off during usage(%) | 29 | 21 | 38 |
| Percentage of leakage to a napkin (%) | 55 | 61 | 45 |

With regard to Table 5 and 7,

1) MD/CD*1 : the flexural rigidity (Gurley bending) in the MD direction／the flexural rigidity (Gurley bending) in the CD direction

2) MD/CD*2 : the ration of the maximum tensile elongation in the MD direction／the maximum tensile elongation in the CD direction

3) Actual usage test*3 : 20 panelists use 5 panels each.

The MD/CD ratios of flexural rigidity of the Examples were 1.1 or 1.2 and thus the flexural rigidity values were substantially the same in the longitudinal direction and the lateral direction, and the flexural rigidity values were in the range from 27 to 110 mg. In regard to the results of the actual usage test with the Examples, the percentage of persons complaining of a foreign-object sensation was no more than 25% and the percentage of pads that fell off was no more than 21% and these were both lower than those of the Comparative Examples. It was thus demonstrated that while an interlabial pad is fitted on, the chances of the absorbent body becoming lopsided in one direction and the bulk of the absorbent body changing at parts to cause a foreign-object sensation are low and that it is difficult for the pad to fall off. It was also shown that by differing the arrangements of the fiber aggregates at the part corresponding to the upper region and at the part corresponding to the lower region of the absorbent body, the percentage of fall-off and the percentage of leakage of menstrual blood are reduced.

Also the interlabial pads used in Example 4 and Example 5 were low in the percentage of leakage of menstrual blood in comparison to Example 1. This is considered to be due to the use of pulp having a short fiber length as a part of the fibers that form the absorbent body, and since the spatial shapes between fibers do not deform readily in this case, the menstrual blood captured between fibers was not discharged readily.

In comparison to Example 1 and Example 5, the probability of complaint of a foreign-body sensation was high with the interlabial pad of Example 4. Whereas the tensile elongation is substantially equal at the upper region and the lower region in the interlabial pad of Example 4, the tensile elongation of the upper region is high in the interlabial pads of Example 1 and Example 5. The above result is thus considered to be due to Example 1 and Example 5 being better in the property of following variations in the movements of the vestibular floor.

As the flexural rigidity of each interlabial pad of each Example that was measured here, the flexural rigidity of the absorbent body which takes up a large part of the total weight of an interlabial pad is used. This is because though the flexural rigidity of an interlabial pad as a whole is influenced by the flexural rigidity of the cover member that encloses the absorbent body and the method of joining the cover member and the absorbent body, it was found by the following measurement that the influence of the flexural rigidity of the absorbent body makes up a large part of the influences on the flexural rigidity of an interlabial pad as a whole in comparison to the influence of these factors.

That is, the influence of whether or not a cover member exists and the influence of the joining method on the overall flexural rigidity of an interlabial pad was measured for the interlabial pad (trade name: "Envive") of PG Corp. and the interlabial pad (trade name: "INSYNC") of A-Fem Medical Corporation, which were indicated as Comparative Examples of the Examples. The measurement results are shown in Table 8. Table 4 shows the results of measuring the flexural rigidities of entire interlabial pads and just the absorbent bodies and the arrangements of the test pieces.

EP 1 598 038 A1

As shown in Table 8, the influence of whether or not a cover member exists and the influence of the joining

[Table 8]

| | | Comparative Example1 (Enviwe) | | Comparative Example2 (INSYNC) | |
| --- | --- | --- | --- | --- | --- |
| | | Interlabial pad | Absorbent body | Interlabial pad | Absorbent body |
| | Material | Chemical bonding nonwoven fabric | — | Point bonding nonwoven fabric | — |
| | Specific weight (g/m²) | 25 | — | 20 | — |
| | Material | Fiber aggregate (Cellulose) | Fiber aggregate (Cellulose) | Fiber aggregate (Cellulose) | Fiber aggregate (Cellulose) |
| | Specific weight (g/m²) | 360 | 360 | 580 | 580 |
| | Material | PBS film | — | Point bonding nonwoven fabric | — |
| | Specific weight (g/m²) | — | — | 20 | — |
| | MD direction (mg) | 35 | 33 | 375 | 368 |
| | CD direction (mg) | 14 | 14 | 135 | 124 |
| | MD/CD*1 | 2.5 | 2.4 | 2.8 | 3.0 |

method on the overall flexural rigidity of an interlabial pad are only no more than 10%. It can thus be said that what defines the flexural rigidity of an absorbent body is equivalent to what defines the flexural rigidity of an interlabial pad as a whole.

The respective measurement methods and the method of preparing artificial menstrual blood shall now be described.

[Measurement of the Apparent Density of an Absorbent Body]

1) The cross-sectional area of an absorbent body is measured at a part extending 5 mm each to the right and left (total of 10 mm) from the longitudinal central line. Though the method of measuring the cross-sectional area of the absorbent body is not restricted as long as the measurement can be made in a no-load state, here, the cross-sectional area was measured using a digital microscope (VH-6200, made by Keyence Corp.). In a case where the absorbent body does not have a flat shape, the measurement was made upon spreading to a flat shape without affecting the cross-sectional area. Cases where the absorbent body does not have a flat shape include, for example, the case where the absorbent body is folded, the case where the absorbent body is wavy in its entirety due to embossing, etc. When the absorbent body has two layers, the cross-sectional area of each layer is measured as it is.

2) Next, a part of the same position that extends 5 mm each to the right and left (total of 10 mm) from the longitudinal direction central line and 5 mm each to the front and rear (total of 10 mm) is cut from a separate sample.

3) The weight of the sample of 2) which was cut to a size of 10 mm $\times$ 10 mm is measured. Here, if the respective layers are integrated by an adhesive agent, embossing, etc., the layers are separated so as not to give rise to a change of weight and the weight of the respective layers are measured in the same manner.

4) The cross-sectional area determined for the no-load state by the measurement of 1), and the area of 10 mm $\times$ 10 mm are multiplied to determine the volume.

5) Values of the weight measured in the step of 3) divided by the volume obtained in the step of 4) are determined to determine the apparent density for N = 10.

[Measurement of the Flexural Rigidity of an Interlabial Pad]

The objects of the measurement are to measure the rigidities of the respective parts of an interlabial pad and to measure the repulsive force directed towards both the left and right outer directions of the interlabial pad.

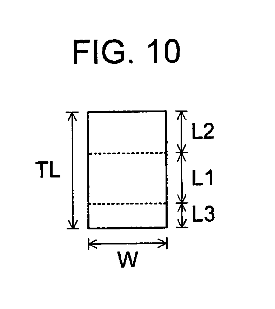

As test pieces, ten interlabial pads are prepared and the size of the measured part is set to be a width W of 0.8 inches (20 mm) and a length of 0.5 inches (13 mm) as shown in Fig. 10. A chuck part of a length L2 of 0.43 inches (10.7 mm) and a part of a length L3 of 0.24 inches (6.3 mm) set onto a pendulum are provided at the respective ends in the longitudinal direction, and the length TL of the entire test piece is set to 1.2 inches (30 mm).

<Measurement Procedure>

1) The above-described test piece is set as follows in a Gurley tester (Gurley's Stiffness Tester, made by Yasuda Seiki Seisakusho., Ltd.) as follows. The chuck part taking up approximately 0.46 inches (10.7 mm) of the upper end in the longitudinal direction of the test piece is set in a chuck, and the part set onto a pendulum which takes up 0.24 inches (6.3 mm) of the lower end is set onto the pendulum.

2) An auxiliary weight is then attached so that reading will fall between 3 and 6.

3) A switch is pressed and the scale at the instant at which the rotating rod of the pendulum separates from the test piece is read.

4) This measurement is repeated five times for the left and right sides.

<Calculation of the Result>

The average value for the left and right sides are calculated by the calculation equation of the following Equation 1.

[Equation 1]

$$\text{Bending resistance (mg)} = (\text{Left} + \text{Right})/2 \times (①\times 1 + ②\times 2 + ③\times 4)/5$$

$$\times (L^2/W) \times 9.88$$

In the above, ① is the weight (g) of a weight which is put into a hole positioned at 1 inch position, ② is the weight (g) of a weight which is put into a hole positioned at 2 inch position, and ③ is the weight (g) of a weight which is put into a hole positioned at 1 inch position. W is the width dimension (in units of inches) of the test piece and L is the length (in units of inches) of the test piece between the chuck and the pendulum.

[Measurement of the Absorption Amount and Absorption Percentage of an Absorbent Body]

The object is to measure the absorption amount (absorption capacity) and absorption percentage of an absorbent body.
As test pieces, 10 absorbent bodies, each with a length of 100 mm each in the longitudinal direction and the lateral direction, are used.

<Measurement Procedure>

1) The weight of each test piece is measured with a scale. This weight is indicated as ④.
2) Artificial menstrual blood is placed in a plastic container until the depth of the artificial menstrual blood is 10 mm or more.
3) The test piece is set so as to be completely immersed in the artificial menstrual blood.
4) The test piece is then left as it is for 3 minutes (environment: temperature 20° C / humidity 60%).
5) After 3 minutes, the sample is pulled out and its weight is measured with a scale. This weight is indicated as ⑤.

<Calculation of Results>

The absorption amount and absorption percentage are indicated by the following Equation 2 and Equation 3.

[Equation 2]

$$\text{Absorption amount (absorption capacity)} = \text{Weight (g) of } ⑤ - \text{Weight (g)}$$

$$\text{of } ④$$

[Equation 3]

$$\text{Absorption percentage} = ((\text{Weight (g) of } ⑤ - \text{Weight (g) of } ④) / \text{Weight}$$

$$\text{(g) of } ④) \times 100$$

[Preparation of Artificial Menstrual Blood]

<Reagents Used>

1) Sodium carboxymethylcellulose (NaCMC) (Wako Pure Chemical Industries, Ltd., Chemical Grade 039-01335)
2) Glycerin (Wako Pure Chemical Industries, Ltd., Wako Primary Grade 072-00621)
3) Sodium chloride (Wako Pure Chemical Industries, Ltd., Special Reagent Grade 191-01665)
4) Sodium bicarbonate (Wako Pure Chemical Industries, Ltd., Wako Primary Grade 198-01315)
5) Red food dye preparation (Koyo Products Co., Ltd., No. 102, Red No. 102)
6) Red food dye preparation (Koyo Products Co., Ltd., No. 2, Red No. 2)
7) Yellow food dye preparation (Koyo Products Co., Ltd., No. 5, Yellow No. 5)

<Preparation Method>

1) Using two graduated cylinders, 3 liters of ion-exchanged water and 1 liter of ion-exchanged water, both prepared using a pure water producing device (Yamato Scientific Co., Ltd., Pure Water Producing Device, Model WG220), are measured out and placed respectively in separate plastic containers.
2) 320g (318g to 322g) of glycerin are placed in yet another plastic container, 32.0g (31.7g to 32.3g) of sodium carboxymethylcellulose are added, and mixed by stirring for 10 minutes with a stirrer (Hsiang Tai Machinery Industry Co;, Ltd., Model DC-2E). In this process, a rotating chip is made to rotate near the bottom of the container and stirring is performed with the rotation speed setting being set to "6" (approximately 600 rpm).
3) While stirring the 3 liters of ion-exchanged water placed in a plastic container using the abovementioned stirrer, the liquid obtained in 2) is added by small amounts at a time. In this process, the chip is made to rotate near the bottom of the container and stirring is performed with the rotation speed setting being set to "10" (approximately 1100 rpm).
4) So as not leave any of the glycerin and sodium carboxymethylcellulose in the plastic container, the entirety is added by rinsing with the 1 liter of ion-exchanged water prepared in the other plastic container.
5) While mixing further, 40g of sodium chloride and 16g of sodium bicarbonate are added by small amounts at a time.
6) A lid is placed to prevent the reagents from splashing out and stirring is performed for approximately 3 hours.
7) While stirring 32g of Red No. 102, 8g of Red No. 2, and 8g of Yellow No. 5 are added and stirring is performed for approximately 1 hour thereafter.
8) Using a rotational viscometer (Shibaura Systems Co., Ltd., Vismetron, Model VGA-4), the liquid obtained in 7) is checked to have a viscosity in the range of 22 to 26mPa·s.

According to the present invention, since the longer the average fiber length of the fibers that form a fiber aggregate, the more readily the fibers become entangled with each other, even when the vestibular floor is stretched, the movement can be followed without the fiber aggregate separating. Also, since the fibers are oriented randomly and the absorbent body will not become lopsided towards one direction readily, the absorbent body can be prevented from becoming separated or lopsided due to movements causing the bulk of the absorbent body to become different at parts and the area of contact with the inner labial walls to decrease while the interlabial pad is fitted on and the interlabial pad can be prevented from falling off.

Also, the interlabial pad has a bendability by making the flexural rigidity of the absorbent body no more than 130 mg, and a wearer is made unlikely to feel a foreign-object sensation. Furthermore, the absorbent body can be prevented from becoming lopsided readily and causing the interlabial pad to fall off while the interlabial pad is fitted on by making the flexural rigidity no less than 25 mg, and furthermore since the interlabial pad can maintain its shape without sagging when the interlabial pad is guided between interlabial pad in the fitting process, a user is enabled to fit on the interlabial pad without fail at the intended location and without the interlabial pad becoming lopsided.

Also, by disposing a high tensile elongation fiber aggregate at an upper region and a low tensile elongation fiber aggregate at a lower region of an absorbent body, the absorbent body at the upper region that contacts the vestibular floor is enabled to stretch and follow the movements of a user. The absorbent body is thus made more unlikely to irritate, especially the hypersensitive vestibular floor and the likelihood of making a wearer feel a foreign-object sensation is reduced. Also, since the flexibility among fibers is restrained at the absorbent body at the lower region and the spatial shapes between fibers are thereby made unlikely to vary regardless of variations in the movements of the body, the menstrual blood that has been captured once between the fibers can be kept held and leakage of menstrual blood is restrained.

**Claims**

1. An interlabial pad comprising:

   an absorbent body absorbing liquid; and
   a cover body covering the absorbent body in an enclosing manner;

   wherein the absorbent body comprises a fiber aggregate in which fiber directions are oriented randomly; and
   the fiber aggregate has a flexural rigidity as Gurley bending resistance being in a range from 25 mg to 130 mg and a ratio of flexural rigidities in two mutually orthogonal directions being in a range from 0.5 to 2.0.

2. The interlabial pad according to Claim 1;
   wherein flexural rigidities in two mutually orthogonal directions of the fiber aggregate are substantially the same.

3. The interlabial pad according to Claim 1 or 2;
   wherein the absorbent body is formed by layering the fiber aggregate and another fiber aggregate that differ each other in tensile elongation; and
   one of the fiber aggregates which is positioned at a vestibular floor side when the interlabial pad is fitted between labia has a higher tensile elongation than that of the other fiber aggregate which is positioned at a side opposite to the vestibular floor side.

4. The interlabial pad according to Claim 3;
   wherein the fiber aggregate positioned at the vestibular floor side has a tensile elongation of 60% or more than that in a dry state even in a wet state in which liquids are absorbed.

5. The interlabial pad according to Claim 3 or 4;
   wherein the fiber aggregate positioned at the side opposite to the vestibular floor side comprises another fiber aggregate that differ in tensile elongation; and
   one of the fiber aggregates which is positioned at the vestibular floor side has a higher tensile elongation than that of the other fiber aggregate which is positioned at the side opposite to the vestibular floor side.

6. The interlabial pad according to Claim 1 or 2;
   wherein the interlabial pad is a substantially planar interlabial pad; and
   the cover body that covers the absorbent body comprises a liquid permeable surface side sheet and a liquid impermeable back face sheet; and
   wherein the absorbent body is formed by layering the fiber aggregate and another fiber aggregate that differ each other in tensile elongation; and
   one of the fiber aggregates which is positioned at the vestibular floor side has a higher tensile elongation than that of the other fiber aggregate which is positioned at the side opposite to the vestibular floor side.

7. The interlabial pad according to Claim 6;
   wherein a proportion of the fiber aggregate having the higher tensile elongation and a proportion of the fiber aggregate having the lower tensile elongation are substantially the same in the thickness direction of the absorbent body.

8. The interlabial pad according to Claim 6;
   wherein a proportion of the fiber aggregate having the higher tensile elongation is larger than a proportion of the fiber aggregate having the lower tensile elongation in the thickness direction of the absorbent body at a vicinity of a longitudinal direction central line.

9. The interlabial pad according to Claim 8,
   wherein the absorbent body comprises the fiber aggregate having the higher tensile elongation at outer peripheral parts and being positioned the entire thickness direction.

**10.** The interlabial pad according to any one of Claims 6 to 9,
wherein the tensile elongation of the fiber aggregate having the higher tensile elongation is maintained at 60% or more than that in the dry state even in the wet state in which liquids are absorbed.

**11.** The interlabial pad according to any one of Claims 6 to 10;
wherein a dividing region which divides the absorbent body is provided at least substantially along the longitudinal direction central line at a rear of the absorbent body.

# FIG. 1

# FIG. 2

# FIG. 3

q

10a

11

14b

r

14a

12a

A

Y

B

# FIG. 4

q

10b

11

14d

r

14c

14d

12b

A

B

Y

C

# FIG. 5 A

# FIG. 5 B

# FIG. 6 A

# FIG. 6 B

# FIG. 7 A

20b

L'

L

242b

242a

222

21a

21b

21

FE · E · M · E · FE

# FIG. 7 B

q

r

20b

21a

242b

242a

222

21b

A

B

C

# FIG. 8 A

L'

20c

243b
243a

223

21a
} 21
21b

FE  E  M  E  FE

# FIG. 8 B

q

20c

r

21a

243b

243a

223

A

B

C

21b

# FIG. 9 A

20d
26
244b
244a
224
21a
21b
21

FE  E  M  E  FE

# FIG. 9 B

q
r
26
21a
244b
244a
244
21b

A
B
C

# FIG. 10

## INTERNATIONAL SEARCH REPORT

International application No.

PCT/JP2004/001729

| A. CLASSIFICATION OF SUBJECT MATTER |
|---|
| Int.Cl$^7$ A61F13/538, 13/472, 13/534, 13/535 |

According to International Patent Classification (IPC) or to both national classification and IPC

| B. FIELDS SEARCHED |
|---|
| Minimum documentation searched (classification system followed by classification symbols)<br>Int.Cl$^7$ A61F13/15-13/84 |

Documentation searched other than minimum documentation to the extent that such documents are included in the fields searched

| Jitsuyo Shinan Koho | 1926–1996 | Toroku Jitsuyo Shinan Koho | 1994–2004 |
|---|---|---|---|
| Kokai Jitsuyo Shinan Koho | 1971–2004 | Jitsuyo Shinan Toroku Koho | 1996–2004 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 10-216172 A (Kao Corp.),<br>18 August, 1998 (18.08.98),<br>Par. Nos. [0018], [0027] to [0031]; Fig. 4<br>& EP 858791 A | 1-4,6-10<br>11 |
| Y | JP 2001-507597 A (The Procter & Gamble Co.),<br>12 June, 2001 (12.06.01),<br>Pages 21 to 22; Fig. 5<br>& WO 98/29078 A | 11 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>11 March, 2004 (11.03.04) | Date of mailing of the international search report<br>30 March, 2004 (30.03.04) |
|---|---|
| Name and mailing address of the ISA/<br>Japanese Patent Office | Authorized officer |
| Facsimile No. | Telephone No. |

Form PCT/ISA/210 (second sheet) (July 1998)

EP 1 598 038 A1

**INTERNATIONAL SEARCH REPORT**

| International application No. |
| --- |
| PCT/JP2004/001729 |

---

**Box I    Observations where certain claims were found unsearchable (Continuation of item 2 of first sheet)**

This international search report has not been established in respect of certain claims under Article 17(2)(a) for the following reasons:

1. ☐ Claims Nos.:

   because they relate to subject matter not required to be searched by this Authority, namely:

2. ☒ Claims Nos.:  5

   because they relate to parts of the international application that do not comply with the prescribed requirements to such an extent that no meaningful international search can be carried out, specifically:
   The description in Claim 5 indicates that the tensile elongation of the portion of a fiber complex positioned on a vestibule floor side among the portion of the fiber complex on the opposite side of the side vestibule floor side is higher than that of the portion (continued to extra sheet)

3. ☐ Claims Nos.:

   because they are dependent claims and are not drafted in accordance with the second and third sentences of Rule 6.4(a).

---

**Box II    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1998)

**INTERNATIONAL SEARCH REPORT**

International application No.

PCT/JP2004/001729

<u>Continuation of Box No.I-2 of continuation of first sheet(1)</u>

thereof positioned at an outermost position on the opposite side of the vestibule floor side.   However, though the inverse structure of the fiber complex positioned on the opposite side of the vestibule floor side is described in the specification, the constitution described in Claim 5 is not described nor suggested in the specification.

Form PCT/ISA/210 (extra sheet) (July 1998)